(19) Europäisches Patentamt / European Patent Office / Office européen des brevets

(11) EP 4 434 468 A1

(12) EUROPEAN PATENT APPLICATION
published in accordance with Art. 153(4) EPC

(43) Date of publication:
**25.09.2024 Bulletin 2024/39**

(21) Application number: **22895499.6**

(22) Date of filing: **09.11.2022**

(51) International Patent Classification (IPC):
***A61B 8/00*** (2006.01) ***A61B 8/12*** (2006.01)
***A61B 8/13*** (2006.01) ***C08K 9/00*** (2006.01)
***C08L 83/04*** (2006.01) ***C08L 83/05*** (2006.01)
***C08L 83/07*** (2006.01)

(52) Cooperative Patent Classification (CPC):
**G10K 11/30; A61B 8/00; A61B 8/12; A61B 8/13; A61B 8/4281; A61B 8/4444; C08K 9/00; C08L 83/04**

(86) International application number:
**PCT/JP2022/041674**

(87) International publication number:
**WO 2023/090217 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.11.2021 JP 2021186567**

(71) Applicant: **FUJIFILM Corporation**
**Tokyo 106-8620 (JP)**

(72) Inventor: **NAKAI, Yoshihiro**
**Ashigarakami-gun, Kanagawa 258-8577 (JP)**

(74) Representative: **HGF**
**HGF Limited**
**1 City Walk**
**Leeds LS11 9DX (GB)**

# (54) COMPOSITION FOR ACOUSTIC LENS, ACOUSTIC LENS, ACOUSTIC WAVE PROBE, ULTRASONIC PROBE, ACOUSTIC WAVE MEASURING DEVICE, ULTRASOUND DIAGNOSTIC DEVICE, PHOTOACOUSTIC WAVE MEASURING DEVICE, ULTRASONIC ENDOSCOPE, AND METHOD FOR PRODUCING ACOUSTIC WAVE PROBE

(57) Provided are a composition for an acoustic lens, containing the following components (A) to (D), an acoustic lens, an acoustic wave probe, an ultrasound probe, an acoustic wave measurement apparatus, an ultrasound diagnostic apparatus, a photoacoustic wave measurement apparatus, an ultrasonic endoscope, and a method for manufacturing an acoustic wave probe.
(A) a linear polysiloxane having a vinyl group,
(B) a linear polysiloxane having two or more Si-H groups in a molecular chain,
(C) a polysiloxane resin, and
(D) zinc oxide particles having an average primary particle diameter of more than 10 nm and less than 300 nm, and surface-treated with at least one surface treatment agent of a silane compound, an aluminum alkoxide compound, a zirconium alkoxide compound, or a titanium alkoxide compound,
provided that the silane compound has at least one of a hydroxy group directly bonded to a silicon atom or an alkoxy group directly bonded to the silicon atom.



Processed by Luminess, 75001 PARIS (FR)

## Description

### BACKGROUND OF THE INVENTION

1. Field of the Invention

The present invention relates to a composition for an acoustic lens, an acoustic lens, an acoustic wave probe, an ultrasound probe, an acoustic wave measurement apparatus, an ultrasound diagnostic apparatus, a photoacoustic wave measurement apparatus, an ultrasonic endoscope, and a method for manufacturing an acoustic wave probe.

2. Description of the Related Art

In an acoustic wave measurement apparatus, an acoustic wave probe is used which irradiates a test object or site (hereinafter, also referred to as "test object or the like") with an acoustic wave, receives a reflected wave (echo) therefrom, and outputs a signal. The electric signal which is converted from the reflected wave received by this acoustic wave probe is displayed as an image. As a result, an inside of the test object is visualized and observed.

As the acoustic wave, an ultrasonic wave, a photoacoustic wave, or the like having an appropriate frequency is selected according to the test object or the like or the measurement conditions or the like.

For example, an ultrasound diagnostic apparatus transmits an ultrasonic wave to the inside of the test object, receives the ultrasonic wave reflected by tissues inside the test object, and displays the received ultrasonic wave as an image. A photoacoustic wave measurement apparatus receives an acoustic wave radiated from the inside of the test object due to a photoacoustic effect, and displays the received acoustic wave as an image. The photoacoustic effect is a phenomenon in which an acoustic wave (typically, an ultrasonic wave) is generated through thermal expansion after a test object absorbs an electromagnetic wave to generate heat in a case where the test object is irradiated with an electromagnetic wave pulse of visible light, near infrared light, microwave, or the like.

An acoustic wave measurement apparatus equipped with an acoustic wave probe is used not only for examining an inside of a body, such as the abdomen and heart, but also for examining tissues near a body surface such as the mammary gland, thyroid gland, peripheral blood vessels, musculoskeletal system, nerves, and skin. The tissues near the body surface have a fine structure, and a high-resolution examination image is required.

Generally, the resolution of an acoustic wave image increases as a frequency of the acoustic wave increases. Furthermore, by lowering an acoustic velocity of an acoustic lens constituting the acoustic wave probe, it is possible to shorten a focal length and to obtain a higher resolution image of the tissues near the body surface. That is, by lowering the acoustic velocity of the acoustic lens, it is possible to obtain more accurate information with regard to living tissues near the body surface.

Since the acoustic wave probe performs transmission and reception of acoustic waves by being rubbed against the body, peeling between the acoustic lens and an acoustic matching layer may occur due to repeated use, and this peeling may cause a shift in focus of an acoustic wave image. The acoustic matching layer has a configuration in which an epoxy resin cured substance is generally used on the acoustic lens side (a portion in contact with the acoustic lens). Therefore, it is necessary for the acoustic lens to have a characteristic of being difficult to peel off from the epoxy resin cured substance constituting the acoustic matching layer.

For example, WO2021/044823A discloses a composition for an acoustic lens, which contains zinc oxide surface-treated with a surface treatment agent including at least one of an aminosilane compound, a mercaptosilane compound, an isocyanatosilane compound, a thiocyanatosilane compound, an aluminum alkoxide compound, a zirconium alkoxide compound, or a titanium alkoxide compound, a polysiloxane having a vinyl group, and a polysiloxane having two or more Si-H groups in a molecular chain. According to the technique disclosed in WO2021/044823A, by using the above-described composition for an acoustic lens, the acoustic lens to be obtained can be made to have a low acoustic velocity, and the acoustic lens is difficult to peel off from the acoustic matching layer and has excellent durability against body fluid.

### SUMMARY OF THE INVENTION

In recent years, as a sterilization treatment of the acoustic lens, a sterilization treatment using ozone water obtained by dissolving ozone ($O_3$) in water has been performed. However, the ozone water generates a strong active species such as a hydroxyl radical, and an oxidation power thereof is stronger than that of a hydrogen peroxide gas. Therefore, in a case where the ozone water intrudes into the acoustic matching layer from the acoustic lens, the ozone water causes the acoustic lens to peel off from the acoustic matching layer.

An object of the present invention is to provide a composition for an acoustic lens, with which an acoustic lens having a low acoustic velocity, being difficult to peel off from an acoustic matching layer, and being unlikely to have a reduced adhesive force with the acoustic matching layer even in a strong sterilization treatment such as ozone water

can be realized, and to provide an acoustic lens obtained by curing the composition.

Another object of the present invention is to provide an acoustic wave probe, an ultrasound probe, an acoustic wave measurement apparatus, an ultrasound diagnostic apparatus, a photoacoustic wave measurement apparatus, and an ultrasonic endoscope, as well as a method for manufacturing an acoustic wave probe, each of which has the acoustic lens.

As a result of intensive studies in view of the above-described objects, the present inventors have found that, by subjecting a linear polysiloxane having a vinyl group, a linear polysiloxane having two or more Si-H groups, and a polysiloxane resin to a curing reaction in the presence of zinc oxide particles which have been treated with a specific surface treatment agent, an acoustic velocity of a silicone resin to be obtained can be sufficiently reduced, the obtained silicone resin has excellent adhesive force with an epoxy resin cured sheet, and the obtained silicone resin can sufficiently maintain the adhesive force even in a strong sterilization treatment such as ozone water (has excellent ozone water resistance). The present invention has been completed based on these findings.

The foregoing objects of the present invention have been achieved by the following means.

<1> A composition for an acoustic lens, comprising the following components (A) to (D):

(A) a linear polysiloxane having a vinyl group;
(B) a linear polysiloxane having two or more Si-H groups in a molecular chain;
(C) a polysiloxane resin; and
(D) zinc oxide particles having an average primary particle diameter of more than 10 nm and less than 300 nm, and surface-treated with at least one surface treatment agent of a silane compound, an aluminum alkoxide compound, a zirconium alkoxide compound, or a titanium alkoxide compound,

provided that the silane compound has at least one of a hydroxy group directly bonded to a silicon atom or an alkoxy group directly bonded to the silicon atom.

<2> The composition for an acoustic lens according to <1>,
in which a content of the component (D) is 25 to 70 parts by mass in 100 parts by mass of a total content of the components (A) to (D).

<3> The composition for an acoustic lens according to <1> or <2>,
in which, in 100 parts by mass of a total content of the components (A) to (D), a content of the component (A) is 20 to 70 parts by mass, a content of the component (B) is 0.1 to 20 parts by mass, and a content of the component (C) is 0.1 to 50 parts by mass.

<4> The composition for an acoustic lens according to any one of <1> to <3>,

in which the silane compound includes at least one of an aminosilane compound, a mercaptosilane compound, an isocyanatosilane compound, or a thiocyanatosilane compound,
provided that the aminosilane compound, the mercaptosilane compound, the isocyanatosilane compound, and the thiocyanatosilane compound have at least one of a hydroxy group directly bonded to a silicon atom or an alkoxy group directly bonded to the silicon atom.

<5> The composition for an acoustic lens according to any one of <1> to <4>,
in which a content of the surface treatment agent in the component (D) is 1 to 100 parts by mass with respect to 100 parts by mass of the zinc oxide particles constituting the component (D).

<6> The composition for an acoustic lens according to any one of <1> to <5>,
in which the component (A) has a phenyl group.

<7> The composition for an acoustic lens according to any one of <1> to <6>,
in which a weight-average molecular weight of the component (A) is 20,000 to 200,000.

<8> The composition for an acoustic lens according to <7>,
in which the weight-average molecular weight is 40,000 to 150,000.

<9> The composition for an acoustic lens according to any one of <1> to <8>,

in which the component (C) is a polysiloxane resin which includes an $R_3SiO_{1/2}$ unit and an $SiO_{4/2}$ unit and has at least two vinyl groups in one molecule, and a molar ratio of the $R_3SiO_{1/2}$ unit to the $SiO_{4/2}$ unit is 0.6 to 1.2, provided that R represents a monovalent hydrocarbon group.

<10> The composition for an acoustic lens according to <9>,
in which the component (C) consists of the $R_3SiO_{1/2}$ unit and the $SiO_{4/2}$ unit.

<11> The composition for an acoustic lens according to any one of <1> to <10>,

in which the average primary particle diameter of the zinc oxide particles constituting the component (D) is 10 to 200 nm.

<12> The composition for an acoustic lens according to any one of <1> to <11>, further comprising:
at least one of platinum or a platinum compound in an amount of 0.00001 to 0.01 parts by mass with respect to 100 parts by mass of a total content of the components (A) to (D).

<13> An acoustic lens obtained by curing the composition for an acoustic lens according to any one of <1> to <12>.

<14> An acoustic wave probe comprising:
the acoustic lens according to <13>.

<15> An ultrasound probe comprising:
the acoustic lens according to <13>.

<16> An acoustic wave measurement apparatus comprising:
the acoustic wave probe according to <14>.

<17> An ultrasound diagnostic apparatus comprising:
the acoustic wave probe according to <14>.

<18> A photoacoustic wave measurement apparatus comprising:
the acoustic lens according to <13>.

<19> An ultrasonic endoscope comprising:
the acoustic lens according to <13>.

<20> A method for manufacturing an acoustic wave probe, comprising:
forming an acoustic lens using the composition for an acoustic lens according to any one of <1> to <12>.

In the description of the present specification, a "metal alkoxide compound (for example, an aluminum alkoxide compound, a zirconium alkoxide compound, a titanium alkoxide compound, or the like which will be described later)" means a compound having a structure in which at least one alkoxy group is bonded to a metal atom. The alkoxy group may have a substituent. The substituent may be monovalent or divalent (for example, an alkylidene group). In addition, two alkoxy groups bonded to one metal atom may be bonded to each other to form a ring.

In the description of the present specification, unless otherwise specified, in a case where a plurality of groups having the same reference numerals are present in the general formula representing a compound, the groups may be the same or different from each other, and the group specified by each group (for example, an alkyl group) may further have a substituent. In addition, the "Si-H group" means a group having three bonding sites on the silicon atom, and the description of these bonding sites is omitted to simplify the notation.

In addition, the expression "to" in the present specification is used to mean that numerical values described before and after "to" are included as a lower limit value and an upper limit value, respectively.

With the composition for an acoustic lens according to the aspect of the present invention, an acoustic lens having a low acoustic velocity, being difficult to peel off from an acoustic matching layer, and being unlikely to have a reduced adhesive force with the acoustic matching layer even in a strong sterilization treatment such as ozone water can be realized.

In addition, the acoustic lens according to the aspect of the present invention has a low acoustic velocity, is difficult to peel off from an acoustic matching layer, and is unlikely to have a reduced adhesive force with the acoustic matching layer even in a strong sterilization treatment such as ozone water.

In addition, the acoustic wave probe, the ultrasound probe, the acoustic wave measurement apparatus, the ultrasound diagnostic apparatus, the photoacoustic wave measurement apparatus, and the ultrasonic endoscope according to the aspects of the present invention include an acoustic lens manufactured using the composition for an acoustic lens, having the above-described excellent performance.

In addition, according to the method for manufacturing an acoustic wave probe according to the aspect of the present invention, an acoustic wave probe including the above-described acoustic lens can be obtained.

## BRIEF DESCRIPTION OF THE DRAWINGS

Fig. 1 is a perspective view of an example of a convex type ultrasound probe which is an aspect of an acoustic wave probe.

## DESCRIPTION OF THE PREFERRED EMBODIMENTS

<<Composition for an acoustic lens>>

The composition for an acoustic lens (hereinafter, also simply referred to as a composition) according to the embodiment of the present invention contains the following components (A) to (D).

(A) a linear polysiloxane having a vinyl group (component (A))
(B) a linear polysiloxane having two or more Si-H groups in a molecular chain (component (B))
(C) a polysiloxane resin (component (C))
(D) zinc oxide particles having an average primary particle diameter of more than 10 nm and less than 300 nm, and surface-treated with at least one surface treatment agent of a silane compound, an aluminum alkoxide compound, a zirconium alkoxide compound, or a titanium alkoxide compound (component (D))

The silane compound has at least one of a hydroxy group directly bonded to a silicon atom or an alkoxy group directly bonded to the silicon atom.

As described above, the composition according to the embodiment of the present invention contains (A) a linear polysiloxane (polyorganosiloxane) having a vinyl group and (B) a linear polysiloxane having two or more Si-H groups in a molecular chain. Here, the (B) linear polysiloxane having two or more Si-H groups in the molecular chain is preferably (B) a linear polyorganosiloxane having two or more Si-H groups in the molecular chain.

Therefore, it is preferable that the composition according to the embodiment of the present invention contains at least the component (A), (B) the polyorganosiloxane having two or more Si-H groups in the molecular chain (component (B)), the component (C), and the component (D).

The acoustic lens according to the embodiment of the present invention, which is obtained by curing the composition according to the embodiment of the present invention having the above-described configuration, has a low acoustic velocity, is difficult to peel off from an acoustic matching layer, and has excellent ozone water resistance. The reasons for these effects are not clear yet, but it is presumed as follows.

In a case where an acoustic lens contains a filler having a large specific gravity, it is presumed that a phase is delayed at a filler interface and the acoustic velocity is decreased due to inertia in a case where an acoustic wave (mainly a longitudinal wave) enters the acoustic lens. Since the component (D) contained in the acoustic lens according to the embodiment of the present invention contains the zinc oxide particles which are surface-treated with a specific surface treatment agent and have a specific average primary particle diameter as a surface-treated product, it is considered that the zinc oxide particles are substantially uniformly dispersed in the lens having a matrix of silicone resin, thereby increasing the phase delay and effectively reducing the acoustic velocity. In addition, as described above, an acoustic matching layer has a configuration in which an epoxy resin cured substance is generally used on the acoustic lens side (a portion in contact with the acoustic lens). The epoxy resin cured substance has a hydroxyl group and is relatively hydrophilic. On the other hand, the matrix of silicone resin, constituting the acoustic lens according to the embodiment of the present invention, is relatively hydrophobic. Since the surface treatment agent (component derived from the surface treatment agent) constituting the component (D) contained in the acoustic lens according to the embodiment of the present invention interacts with a polar group such as a hydroxyl group, included in the epoxy resin cured substance, it is considered that adhesiveness between the hydrophilic resin and the hydrophobic resin, so that it is difficult to peel off the acoustic lens from the acoustic matching layer. In addition, since the component (D) having a specific average primary particle diameter is densely and substantially uniformly dispersed in the matrix of silicone resin, and it is possible to effectively prevent ozone from being transmitted, it is considered that the above-described acoustic lens has excellent ozone water resistance.

From the viewpoint of acoustic velocity, adhesiveness, and ozone water resistance of the acoustic lens, a content of the component (D) is preferably 25 to 70 parts by mass, more preferably 30 to 65 parts by mass, still more preferably 30 to 60 parts by mass, even more preferably 35 to 60 parts by mass, and even still more preferably 35 to 50 parts by mass in 100 parts by mass of the total content of the components (A) to (D). In addition, the content of the component (D) is also preferably 35 to 60 parts by mass and also preferably 40 to 60 parts by mass.

In addition, contents of the components (A) to (C) in 100 parts by mass of the total content of the components (A) to (D) are preferably in the following ranges.

From the viewpoint of acoustic velocity, adhesiveness, and ozone water resistance of the acoustic lens, the content of the component (A) is preferably 20 to 70 parts by mass, more preferably 25 to 65 parts by mass, still more preferably 25 to 60 parts by mass, even more preferably 27 to 57 parts by mass, and even still more preferably 30 to 55 parts by mass in 100 parts by mass of the total content of the components (A) to (D). The content of the component (B) is preferably 0.1 to 20 parts by mass, more preferably 0.2 to 10 parts by mass, still more preferably 0.3 to 6 parts by mass, and even more preferably 0.3 to 2.5 parts by mass. The content of the component (C) is preferably 0.1 to 50 parts by mass, more preferably 2 to 30 parts by mass, still more preferably 5 to 20 parts by mass, even more preferably 6 to 18 parts by mass, and even still more preferably 8 to 18 parts by mass. In addition, the content of the component (C) is also preferably 4 to 16 parts by mass, also preferably 5 to 15 parts by mass, also preferably 6 to 14 parts by mass, and also preferably 7 to 13 parts by mass.

In the following detailed description, the component (A) and the (B) the linear polyorganosiloxane having two or more Si-H groups in the molecular chain (component (B)) will be described as a preferred aspect. However, the present is not limited to the aspects described below.

<(A) Linear polysiloxane having vinyl group>

The component (A) used in the present invention preferably has two or more vinyl groups in a molecular chain thereof.

The component (A) may be, for example, a polysiloxane (a1) having vinyl groups at least at both terminals of a molecular chain thereof (hereinafter, also simply referred to as component (a1)), or a polysiloxane (a2) having at least two $-O-Si(CH_3)_2(CH=CH_2)$ in a molecular chain thereof excluding the terminals (hereinafter, also simply referred to as polysiloxane (a2)). Among these, the polysiloxane (a1) having vinyl groups at least at both terminals of a molecular chain thereof is preferable.

The polysiloxane (a2) is preferably a polysiloxane (a2) in which $-O-Si(CH_3)_2(CH=CH_2)$ is bonded to a Si atom constituting a main chain.

The component (A) is hydrosilylated by the reaction with the component (B), for example, in the presence of a platinum catalyst. A crosslinking structure (cured body) can be formed by this hydrosilylation reaction (addition reaction).

The content of the vinyl group in the component (A) is not particularly limited. From the viewpoint of forming a sufficient network with each component contained in the composition for an acoustic lens, the content of the vinyl group is, for example, preferably 0.01 to 5 mol% and more preferably 0.05 to 2 mol%.

Here, the content of the vinyl group is mol% of a vinyl group-containing siloxane unit in a case where all units constituting the component (A) are set to 100 mol%. One vinyl group-containing siloxane unit has one to three vinyl groups. Among these, the number of vinyl groups is preferably one for one vinyl group-containing siloxane unit. For example, in a case where all Si atoms of a Si-O unit constituting the main chain and a terminal Si have at least one vinyl group, it amounts to 100 mol%.

In addition, the component (A) preferably has a phenyl group, and the content of the phenyl group in the polyorganosiloxane (A) is not particularly limited. From the viewpoint of mechanical strength in a case of being made into an acoustic lens, the content of the phenyl group is, for example, preferably 1 to 80 mol% and more preferably 2 to 40 mol%.

Here, the content of the phenyl group is mol% of a phenyl group-containing siloxane unit in a case where all units constituting the component (A) are set to 100 mol%. One phenyl group-containing siloxane unit has one to three phenyl groups. Among these, the number of phenyl groups is preferably two for one phenyl group-containing siloxane unit. For example, in a case where all Si atoms of a Si-O unit constituting the main chain and a terminal Si have at least one phenyl group, it amounts to 100 mol%.

The "unit" of polysiloxane refers to the Si-O unit constituting the main chain and the terminal Si.

A degree of polymerization and specific gravity are not particularly limited. From the viewpoint of improving the mechanical strength (tear strength) and chemical stability of the obtained acoustic lens, the viscosity of the composition before curing, and the like, the degree of polymerization is preferably 200 to 3,000 and more preferably 400 to 2,000, and the specific gravity is preferably 0.9 to 1.1.

In order to improve the mechanical strength of the acoustic lens and further improve the adhesiveness to the acoustic matching layer, a weight-average molecular weight of the component (A) is preferably 20,000 to 200,000, more preferably 40,000 to 150,000, and still more preferably 45,000 to 120,000.

The weight-average molecular weight can be measured, for example, by using a GPC device HLC-8220 (trade name, manufactured by Tosoh Corporation), toluene (manufactured by FUJIFILM Wako Pure Chemical Corporation) as an eluent, TSKgel G3000HXL + TSKgel G2000HXL (both trade names) as columns, and a differential refractive index (RI) detector under the conditions of a temperature of 23°C and a flow rate of 1 mL/min.

A kinematic viscosity of the component (A) at 25°C is preferably $1 \times 10^{-5}$ to 10 $m^2/s$, more preferably $1 \times 10^{-4}$ to 1 $m^2/s$, and still more preferably $1 \times 10^{-3}$ to 0.5 $m^2/s$.

The kinematic viscosity can be determined by measuring at a temperature of 25°C using a Ubbelohde type viscometer (for example, SU: trade name, manufactured by Sibata Scientific Technology Ltd.) in accordance with JIS Z8803.

The polyorganosiloxane (a1) having vinyl groups at least at both terminals of a molecular chain thereof is preferably a polyorganosiloxane represented by General Formula (A).

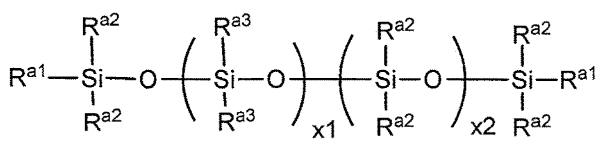 (A)

In General Formula (A), $R^{a1}$ represents a vinyl group, and $R^{a2}$ and $R^{a3}$ each independently represent an alkyl group, a cycloalkyl group, an alkenyl group, or an aryl group. x1 and x2 each independently represent an integer of 1

or more.

The number of carbon atoms in the alkyl group in $R^{a2}$ and $R^{a3}$ is preferably 1 to 10, more preferably 1 to 4, still more preferably 1 or 2, and particularly preferably 1. The alkyl group may be linear or branched, and examples thereof include methyl, ethyl, n-propyl, isopropyl, n-butyl, isobutyl, n-hexyl, n-octyl, 2-ethylhexyl, and n-decyl.

The number of carbon atoms in the cycloalkyl group in $R^{a2}$ and $R^{a3}$ is preferably 3 to 10, more preferably 5 to 10, and still more preferably 5 or 6. In addition, the cycloalkyl group is preferably a 3-membered ring, a 5-membered ring, or a 6-membered ring, and more preferably a 5-membered ring or a 6-membered ring. Examples of the cycloalkyl group include cyclopropyl, cyclopentyl, and cyclohexyl.

The number of carbon atoms in the alkenyl group in $R^{a2}$ and $R^{a3}$ is preferably 2 to 10, more preferably 2 to 4, and still more preferably 2. The alkenyl group may be linear or branched, and examples thereof include vinyl, allyl, and butenyl.

The number of carbon atoms in the aryl group in $R^{a2}$ and $R^{a3}$ is preferably 6 to 12, more preferably 6 to 10, and still more preferably 6 to 8. Examples of the aryl group include phenyl, tolyl, and naphthyl.

The alkyl group, the cycloalkyl group, the alkenyl group, and the aryl group each may have a substituent. Examples of such a substituent include a halogen atom, an alkyl group, a cycloalkyl group, an alkenyl group, an aryl group, an alkoxy group, an aryloxy group, an alkylthio group, an arylthio group, a silyl group, and a cyano group.

Examples of the group having a substituent include a halogenated alkyl group.

$R^{a2}$ and $R^{a3}$ are preferably an alkyl group, an alkenyl group, or an aryl group, more preferably an alkyl group having 1 to 4 carbon atoms, a vinyl group, or a phenyl group, still more preferably a methyl group, a vinyl group, or a phenyl group, and particularly preferably a methyl group or a phenyl group.

Among these, $R^{a2}$ is preferably a methyl group. Among these, $R^{a3}$ is preferably a methyl group, a vinyl group, or a phenyl group, more preferably a methyl group or a phenyl group, and particularly preferably a phenyl group having a large molecular area, in consideration of barrier properties.

x1 is preferably an integer of 200 to 3,000 and more preferably an integer of 400 to 2,000.

x2 is preferably an integer of 1 to 3,000, more preferably an integer of 1 to 1,000, still more preferably an integer of 40 to 1,000, and particularly preferably an integer of 40 to 700.

In addition, as another aspect, x1 is preferably an integer of 1 to 3,000 and more preferably an integer of 5 to 1,000.

In the present invention, repeating units "$-Si(R^{a3})_2-O-$" and "$-Si(R^{a2})_2-O-$" in General Formula (A) may each exist in a block-polymerized form or may be in a form that exists at random.

Examples of the polyorganosiloxane having vinyl groups at least at both terminals of a molecular chain thereof include DMS series (for example, DMS-V31, DMS-V31S15, DMS-V33, DMS-V35, DMS-V35R, DMS-V41, DMS-V42, DMS-V46, DMS-V51, and DMS-V52), PDV series (for example, PDV-0341, PDV-0346, PDV-0535, PDV-0541, PDV-1631, PDV-1635, PDV-1641, and PDV-2335), PMV-9925, PVV-3522, FMV-4031, and EDV-2022, all of which are trade names manufactured by Gelest, Inc.

The DMS-V31S15 is pre-formulated with fumed silica, so that no kneading with a special device is required.

The component (A) in the present invention may be used alone or in combination of two or more thereof.

<(B) Linear polysiloxane having two or more Si-H groups in molecular chain>

The component (B) used in the present invention has two or more Si-H groups in a molecular chain thereof. Here, in a case where the component (B) has a "$-SiH_2-$" structure, the number of Si-H groups in the "$-SiH_2-$" structure is counted as two. In addition, in a case where the component (B) has a "$-SiH_3$" structure, the number of Si-H groups in the "$-SiH_3$" structure is counted as three.

By having two or more Si-H groups in the molecular chain, it is possible to crosslink a polyorganosiloxane having at least two polymerizable unsaturated groups.

From the viewpoint of mechanical strength of the acoustic lens and viscosity of the composition before curing, a weight-average molecular weight of the component (B) is preferably 500 to 100,000 and more preferably 1,500 to 50,000.

The component (B) is preferably a polyorganosiloxane represented by General Formula (B).

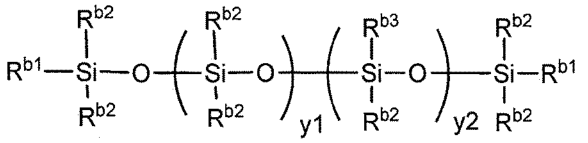

(B)

In General Formula (B), $R^{b1}$ to $R^{b3}$ each independently represent a hydrogen atom, an alkyl group, a cycloalkyl group, an alkenyl group, or an aryl group. y1 and y2 each independently represent an integer of 1 or more. Here, the molecular chain has two or more Si-H groups.

The alkyl group, the cycloalkyl group, the alkenyl group, and the aryl group in $R^{b1}$ to $R^{b3}$, have the same meanings as the alkyl group, the cycloalkyl group, the alkenyl group, and the aryl group in $R^{a2}$ and $R^{a3}$, and preferred aspects thereof are also the same.

$R^{b1}$ to $R^{b3}$ are each preferably a hydrogen atom, an alkyl group, an alkenyl group, or an aryl group, and more preferably a hydrogen atom, an alkyl group having 1 to 4 carbon atoms, a vinyl group, or a phenyl group.

Among these, $R^{b1}$ and $R^{b2}$ are each preferably a hydrogen atom, an alkyl group, an alkenyl group, or an aryl group, more preferably a hydrogen atom or an alkyl group, still more preferably a hydrogen atom or a methyl group, and particularly preferably a methyl group.

$R^{b3}$ is preferably a hydrogen atom, an alkyl group, an alkenyl group, or an aryl group, more preferably a hydrogen atom or an aryl group, still more preferably a hydrogen atom or a phenyl group, and particularly preferably a hydrogen atom.

y1 is preferably an integer of 0 to 2,000, more preferably an integer of 0 to 1,000, and still more preferably an integer of 0 to 30.

y2 is preferably an integer of 1 to 2,000, more preferably an integer of 1 to 1,000, and still more preferably an integer of 1 to 30.

y1 + y2 is preferably an integer of 5 to 2,000, more preferably an integer of 7 to 1,000, still more preferably an integer of 10 to 50, and particularly preferably an integer of 15 to 30.

In the present invention, "$-Si(R^{b2})_2-O-$" and "$-Si(R^{b2})(R^{b3})_2-O-$" in General Formula (B) may each exist in a block-polymerized form in polysiloxane or may be in a form that exists at random.

A combination of $R^{b1}$ to $R^{b3}$ is preferably a combination of $R^{b1}$ being a hydrogen atom or an alkyl group having 1 to 4 carbon atoms, $R^{b2}$ being a hydrogen atom or an alkyl group having 1 to 4 carbon atoms, and $R^{b3}$ being a hydrogen atom or an aryl group; and more preferably a combination of $R^{b1}$ being an alkyl group having 1 to 4 carbon atoms, $R^{b2}$ being an alkyl group having 1 to 4 carbon atoms, and $R^{b3}$ being a hydrogen atom.

In this preferred combination, the content of the hydrosilyl group represented by y2/(y1 + y2) is preferably more than 0.1 and 1.0 or less, and more preferably more than 0.2 and 1.0 or less.

Examples of the component (B) include HMS-064 (MeHSiO: 5 to 7 mol%), HMS-082 (MeHSiO: 7 to 8 mol%), HMS-301 (MeHSiO: 25 to 30 mol%), and HMS-501 (MeHSiO: 50 to 55 mol%) as methylhydrosiloxane-dimethylsiloxane copolymers (trimethylsiloxane terminated), HPM-502 (MeHSiO: 45 to 50 mol%) as a methylhydrosiloxane-phenylmethylsiloxane copolymer, and HMS-991 (MeHSiO: 100 mol%) as a methylhydrosiloxane polymer, all of which are manufactured by Gelest, Inc.

Here, the mol% of MeHSiO has the same meaning as that y2/(y1 + y2) in the preferred combination of $R^{b1}$ to $R^{b3}$ is multiplied by 100.

From the viewpoint of preventing a progress of crosslinking reaction in the molecule, it is preferable that the component (B) does not have a vinyl group.

The component (B) used in the present invention may be used alone or in combination of two or more thereof.

<(C) Polysiloxane resin>

The polysiloxane resin is a polysiloxane compound having a three-dimensional network structure. The component (C) used in the present invention is not particularly limited, but a polysiloxane resin including an $SiO_{4/2}$ unit (Q unit) and an $R_3SiO_{1/2}$ unit (M unit) is preferable. In the M unit, R represents a monovalent hydrocarbon group. R may or may not have a carbon-carbon unsaturated bond. It is preferable that at least one of M units constituting the polysiloxane resin includes a monovalent unsaturated hydrocarbon group having a carbon-carbon unsaturated bond. However, the carbon-carbon unsaturated bond does not include a carbon-carbon double bond in an aromatic ring.

The number of carbon atoms in the above-described monovalent hydrocarbon group not having a carbon-carbon unsaturated bond is preferably 1 to 18, more preferably 1 to 10, and particularly preferably 1 to 8. Specific examples thereof include a linear or branched alkyl group such as methyl, ethyl, propyl, i-propyl, butyl, isobutyl, t-butyl, pentyl, neopentyl, hexyl, cyclohexyl, octyl, nonyl, and decyl; an aryl group such as phenyl, tolyl, xylyl, and naphthyl; an aralkyl group such as benzyl, phenylethyl, and phenylpropyl; and a monovalent group in which a part or all of hydrogen atoms of these groups are substituted with at least one functional group of a halogen atom such as a fluorine atom, a bromine atom, and a chlorine atom, or a cyano group, for example, chloromethyl, chloropropyl, bromoethyl, trifluoropropyl, and cyanoethyl. Among these, methyl is preferable.

The number of carbon atoms in the above-described monovalent unsaturated hydrocarbon group having a carbon-carbon unsaturated bond is preferably 2 to 18, more preferably 2 to 10, still more preferably 2 to 8, and particularly preferably 2 to 6. Examples of the monovalent unsaturated hydrocarbon group having a carbon-carbon unsaturated bond include a linear or branched alkenyl group and a linear or branched alkynyl group, and an alkenyl group is preferable. Specific examples of the monovalent unsaturated hydrocarbon group having a carbon-carbon unsaturated bond include vinyl, allyl, propenyl, i-propenyl, butenyl, hexenyl, cyclohexenyl, and octenyl. Among these, vinyl is preferable.

From the viewpoint of acoustic velocity, adhesiveness, and ozone water resistance of the acoustic lens, a molar

ratio (M/Q) of the $R_3SiO_{1/2}$ unit (M unit) to the $SiO_{4/2}$ unit (Q unit) is preferably 0.1 to 3.0, more preferably 0.3 to 2.5, still more preferably 0.5 to 2.0, even more preferably 0.6 to 1.2, and particularly preferably 0.7 to 1.2. The component (C) may include an $R_2SiO_{2/2}$ unit (D unit) and an $RSiO_{3/2}$ unit (T unit) in the molecule. R in the D unit and the T unit has the same meaning as R in the M unit, and preferred ranges thereof are also the same.

The component (C) preferably has at least two monovalent unsaturated hydrocarbon groups having a carbon-carbon unsaturated bond in one molecule, and a content of the monovalent unsaturated hydrocarbon groups having a carbon-carbon unsaturated bond in the component (C) is preferably $1 \times 10^{-5}$ to $1 \times 10^{-2}$ mol/g and more preferably $1 \times 10^{-4}$ to $2 \times 10^{-3}$ mol/g.

In the composition according to the embodiment of the present invention, a matrix of resin is formed through the component (C) to obtain an overall uniform and dense cured substance (acoustic lens). Since the mechanical strength and ozone water resistance of the cured substance are more excellent, it is preferable that the component (C) consists of the $SiO_{4/2}$ unit (Q unit) and the $R_3SiO_{1/2}$ unit (M unit), and has at least two vinyl groups in one molecule.

It is more preferable that the component (C) consists of one kind of the Q unit ($SiO_{4/2}$ unit) and two kinds of the M units ($R^1_2R^2SiO_{1/2}$ unit and $R^1_3SiO_{1/2}$ unit).

Here, $R^1$ represents the above-described monovalent hydrocarbon group not having a carbon-carbon unsaturated bond, and $R^2$ represents the above-described unsaturated hydrocarbon group.

The component (C) is particularly preferably represented by the following average compositional formula (1).

$$(SiO_{4/2})_b(R^1_2R^2SiO_{1/2})_c(R^1_3SiO_{1/2})_d \qquad \text{Average compositional formula (1)}$$

In the formula, b, c, and d each represent a positive number, $R^1$ represents the above-described monovalent hydrocarbon group not having a carbon-carbon unsaturated bond, and $R^2$ represents the above-described unsaturated hydrocarbon group.

In the present invention, the component (C) is a solid or a viscous liquid at room temperature (25°C). A weight-average molecular weight of the component (C) is not particularly limited, but is preferably a weight-average molecular weight at which a kinematic viscosity of a 50% by mass xylene solution of the component (C) is 0.5 to 10 $mm^2/s$, and more preferably a weight-average molecular weight at which a kinematic viscosity of a 50% by mass xylene solution of the component (C) is 1.0 to 5.0 $mm^2/s$. The above-described kinematic viscosity is a value measured at 25°C using an Ubbelohde-type Ostwald viscometer. The viscosity of the component (C) within the above-described range does not reduce the physical properties of the composition, which is preferable.

The polysiloxane resin is available on the market. For example, VQX-221 (xylene solution) commercially available from Gelest, Inc. can be used.

The component (C) used in the present invention may be used alone or in combination of two or more thereof.

<Zinc oxide particles having average primary particle diameter of more than 10 nm and less than 300 nm, and surface-treated with at least one surface treatment agent of silane compound, aluminum alkoxide compound, zirconium alkoxide compound, or titanium alkoxide compound (component (D))>

The component (D) is zinc oxide particles having an average primary particle diameter (average primary particle diameter in a surface-treated state) of more than 10 nm and less than 300 nm, and surface-treated with at least one surface treatment agent of a silane compound, an aluminum alkoxide compound, a zirconium alkoxide compound, or a titanium alkoxide compound.

Here, the silane compound has at least one of a hydroxy group directly bonded to a silicon atom or an alkoxy group directly bonded to the silicon atom.

From the viewpoint of acoustic velocity, adhesiveness, and ozone water resistance of the acoustic lens, the average primary particle diameter of the zinc oxide particles (zinc oxide particles before being surface-treated (untreated zinc oxide particles)) used in the present invention is preferably 10 to 200 nm, more preferably 10 to 150 nm, still more preferably 10 to 80 nm, and particularly preferably 10 to 50 nm. In addition, the average primary particle diameter of the zinc oxide particles is also preferably 12 to 200 nm, also preferably 15 to 200 nm, also preferably 18 to 150 nm, and also preferably 18 to 100 nm.

In addition, the average primary particle diameter of the component (D) (average primary particle diameter in the surface-treated state) is preferably more than 10 nm and 250 nm or less, more preferably more than 10 nm and 200 nm or less, and still more preferably more than 10 nm and 100 nm or less. In addition, the average primary particle diameter of the component (D) is also preferably 13 to 250 nm, also preferably 16 to 220 nm, also preferably 20 to 200 nm, also preferably 20 to 150 nm, also preferably 20 to 120 nm, and also preferably 20 to 100 nm.

The average primary particle diameter is described in a catalog of a manufacturer of the untreated zinc oxide particles or the surface-treated zinc oxide particles. However, an average primary particle diameter which is not listed in the catalog or the average primary particle diameter or an average primary particle diameter newly manufactured can

be obtained by averaging particle diameters measured by transmission electron microscopy (TEM). That is, the shortest diameter and the longest diameter of one particle in an electron micrograph captured by TEM are measured, and the arithmetic mean value thereof is obtained as a particle diameter of one particle. In the present invention, particle diameters of 300 randomly selected particles are averaged and determined as the average primary particle diameter.

As the zinc oxide particles, commercially available zinc oxide particles can be used, and examples thereof include FINEX series manufactured by Sakai Chemical Industry Co., Ltd. and ZnO-CX manufactured by SUMITOMO OSAKA CEMENT Co., Ltd. (all trade names).

Hereinafter, the surface treatment agent used in the present invention will be specifically described.

(Silane compound)

The silane compound is preferably a silane coupling agent, and preferably includes at least one of an aminosilane compound, a mercaptosilane compound, an isocyanatosilane compound, or a thiocyanatosilane compound.

Here, the aminosilane compound, the mercaptosilane compound, the isocyanatosilane compound, and the thiocyanatosilane compound have at least one of a hydroxy group directly bonded to a silicon atom or an alkoxy group directly bonded to the silicon atom.

-Aminosilane compound-

The aminosilane compound (silane compound having an amino group) is preferably a silane coupling agent having an amino group. It is preferable that the zinc oxide particles surface-treated with the aminosilane compound have an amino group derived from the aminosilane compound.

The aminosilane compound preferably includes at least one compound represented by General Formula (SA).

General Formula (SA) 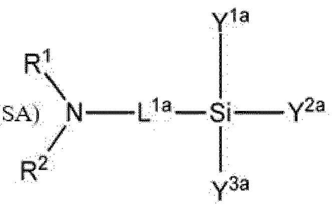

In the formula, $R^1$ and $R^2$ represent a hydrogen atom or a substituent. $L^{1a}$ represents a single bond, an alkylene group, an alkenylene group, an alkynylene group, an arylene group,-O-, -S-, -$NR^a$-, an ester bond, a thioester bond, an amide bond, a thioamide bond or a sulfonyl group, or a divalent group consisting of a combination of two or more of these groups or bonds. $R^a$ represents a hydrogen atom or a substituent. $Y^{1a}$ represents a hydroxy group or an alkoxy group. $Y^{2a}$ and $Y^{3a}$ represent a hydroxy group, an alkoxy group, an alkyl group, or a ketoxime group.

Examples of the substituent that can be used as $R^1$ and $R^2$ include an alkyl group (preferably having 1 to 12 carbon atoms and more preferably 1 to 8 carbon atoms), an alkenyl group (preferably having 2 to 12 carbon atoms and more preferably 2 to 8 carbon atoms), an alkynyl group (preferably having 2 to 12 carbon atoms and more preferably 2 to 8 carbon atoms), and an aryl group (preferably having 6 to 20 carbon atoms and more preferably 6 to 10 carbon atoms). These substituents may further have a substituent, and examples of such a substituent include the above-described substituents mentioned as a substituent that can be used as $R^1$ and $R^2$ and an amino group.

In addition, $R^1$ and $R^2$ may be combined to represent an alkylidene group (preferably having 2 to 12 carbon atoms and more preferably 2 to 8 carbon atoms).

$L^{1a}$ preferably represents an alkylene group, an alkenylene group, an arylene group, -O-, or -$NR^a$-, more preferably an alkylene group, an arylene group, or -$NR^a$-, and still more preferably an alkylene group.

$Y^{1a}$ preferably represents an alkoxy group.

$Y^{2a}$ and $Y^{3a}$ preferably represent a hydroxy group, an alkoxy group, or an alkyl group, and more preferably an alkoxy group or an alkyl group.

The alkylene group that can be used as $L^{1a}$ may be linear, branched, or cyclic. The number of carbon atoms in the alkylene group is preferably 1 to 30, more preferably 1 to 25, still more preferably 1 to 20, and even still more preferably 1 to 15. Specific examples of the alkylene group include methylene, ethylene, propylene, tert-butylene, pentylene, cyclohexylene, heptylene, octylene, nonylene, decylene, and undecylene.

The alkenylene group that can be used as $L^{1a}$ may be linear or branched. The number of carbon atoms in the alkenylene group is preferably 2 to 20, more preferably 2 to 15, still more preferably 2 to 10, and even still more preferably 2 to 6. Specific examples of the alkenylene group include ethenylene and propenylene.

The alkynylene group that can be used as $L^{1a}$ may be linear or branched. The number of carbon atoms in the

alkynylene group is preferably 2 to 20, more preferably 2 to 15, still more preferably 2 to 10, and even more preferably 2 to 6. Specific examples of the alkynylene group include ethynylene and propynylene.

The number of carbon atoms in the arylene group that can be used as $L^{1a}$ is preferably 6 to 20, more preferably 6 to 15, still more preferably 6 to 12, and even still more preferably 6 to 10. Specific examples of the arylene group include phenylene and naphthylene.

Examples of the substituent that can be used as $R^a$ of $-NR^a-$ include an alkyl group (preferably having 1 to 12 carbon atoms and more preferably 1 to 8 carbon atoms), an alkenyl group (preferably having 2 to 12 carbon atoms and more preferably 2 to 8 carbon atoms), an alkynyl group (preferably having 2 to 12 carbon atoms and more preferably 2 to 8 carbon atoms), an aryl group (preferably having 6 to 20 carbon atoms and more preferably 6 to 10 carbon atoms), and a heterocyclic group. A heterocyclic ring constituting the heterocyclic group that can be used as $R^a$ may be a saturated or unsaturated aliphatic heterocyclic ring or aromatic heterocyclic ring, and may be a monocyclic ring or a fused ring. In addition, the heterocyclic ring may also be a bridged ring. Examples of a heteroatom (ring-forming heteroatom) constituting the heterocyclic ring include an oxygen atom, a nitrogen atom, and a sulfur atom. The number of heteroatoms (ring-forming heteroatoms) included in one heterocyclic ring is not particularly limited, but is preferably 1 to 3 and more preferably 1 or 2. The number of carbon atoms constituting the heterocyclic ring (number of ring-forming carbon atoms) is preferably 2 to 10 and more preferably 4 or 5. The heterocyclic ring is preferably a 3- to 7-membered ring, more preferably a 3- to 6-membered ring, and still more preferably a 3- to 5-membered ring. Specific examples of the heterocyclic ring include an epoxy ring, a 3,4-epoxycyclohexane ring, a furan ring, and a thiophene ring.

Examples of $-NR^a-$ include -NH-.

The number of groups or bonds to be combined that constitute a divalent group consisting of a combination of two or more of the above-described groups or the above-described bonds that can be used as $L^{1a}$ (hereinafter, also referred to as a "group consisting of a combination that can be used as $L^{1a}$") is preferably 2 to 8, more preferably 2 to 6, and still more preferably 2 to 4.

In addition, the molecular weight of the group consisting of a combination that can be used as $L^{1a}$ is preferably 20 to 1,000, more preferably 30 to 500, and still more preferably 40 to 200.

Examples of the group consisting of a combination that can be used as $L^{1a}$ include a urea bond, a thiourea bond, a carbamate group, a sulfonamide bond, arylene-alkylene, -O-alkylene, amide bond-alkylene, -S-alkylene, alkylene-O-amide bond-alkylene, alkylene-amide bond-alkylene, alkenylene-amide bond-alkylene, alkylene-ester bond-alkylene, arylene-ester bond-alkylene, -(alkylene-O)-, alkylene-O-(alkylene-O)-alkylene (in which "(alkylene-O)" is a repeating unit), arylene-sulfonyl-O-alkylene, and ester bond-alkylene.

The alkyl group constituting the alkoxy group that can be used as $Y^{1a}$ to $Y^{3a}$ may be linear, branched, or cyclic, and may have a combination of these forms. In the present invention, the alkyl group is preferably a linear alkyl group. The number of carbon atoms in the alkyl group constituting the alkoxy group is preferably 1 to 15, more preferably 1 to 10, still more preferably 1 to 5, and even still more preferably 1 or 2. Specific examples of the alkyl group constituting the alkoxy group include methyl, ethyl, propyl, t-butyl, pentyl, and cyclohexyl.

Examples of the alkyl group that can be used as $Y^{2a}$ and $Y^{3a}$ include an alkyl group that constitutes the alkoxy group that can be used as $Y^{1a}$ to $Y^{3a}$, and a preferred form thereof is also the same as the preferred form of the alkyl group that constitutes the alkoxy group that can be used as $Y^{1a}$ to $Y^{3a}$.

The ketoxime group that can be used as $Y^{2a}$ and $Y^{3a}$ is a substituent having the following structure.

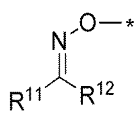

In the above-described structure, $R^{11}$ and $R^{12}$ represent a substituent, and * represents a bonding portion to a silicon atom.

Examples of the substituent that can be used by $R^{11}$ and $R^{12}$ include the substituents in $R^a$, and a preferred form thereof is also the same as the preferred form of the substituent that can be used as $R^a$.

Examples of the ketoxime group include a dimethyl ketoxime group, a methyl ethyl ketoxime group, and a diethyl ketoxime group.

Hereinafter, specific examples of the aminosilane compound used in the present invention will be given, but the present invention is not limited thereto.

3-Aminopropyltrimethoxysilane
3-Aminopropyldimethylmethoxysilane

3-Aminopropylmethyldimethoxysilane
3-Aminopropylmethyldiethoxysilane
3-Aminopropyltriethoxysilane
N-(2-aminoethyl)-3-aminopropylmethyldimethoxysilane
N-(2-aminoethyl)-3-aminopropylmethyldiethoxysilane
N-(2-aminoethyl)-3-aminopropyldimethoxysilane
N-(2-aminoethyl)-3-aminopropyldiethoxysilane
N-(2-aminoethyl)-3-aminopropyltrimethoxysilane
N-(2-aminoethyl)-3-aminopropyltriethoxysilane
3-Methyldimethoxysilyl-N-(1,3-dimethyl-butylidene)propylamine
3-Methyldiethoxysilyl-N-(1,3-dimethyl-butylidene)propylamine
3-Trimethoxysilyl-N-(1,3-dimethyl-butylidene)propylamine
3-Triethoxysilyl-N-(1,3-dimethyl-butylidene)propylamine
N-phenyl-3-aminopropylmethyldiethoxysilane
N-Phenyl-3-aminopropyltrimethoxysilane
N-Phenyl-3-aminopropyltriethoxysilane
N-(vinylbenzyl)-2-aminoethyl-3-aminopropyltrimethoxysilane

-Mercaptosilane compound-

The mercaptosilane compound (silane compound having a mercapto group (sulfanyl group)) is preferably a silane coupling agent having a mercapto group. The zinc oxide particles surface-treated with the mercaptosilane compound preferably have a mercapto group derived from the mercaptosilane compound.

The mercaptosilane compound preferably includes at least one compound represented by General Formula (SM).

General Formula (SM) 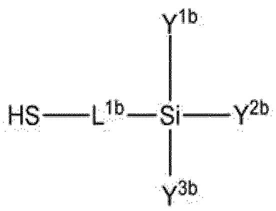

$L^{1b}$, $Y^{1b}$, $Y^{2b}$, and $Y^{3b}$ have the same definition as $L^{1a}$, $Y^{1a}$, $Y^{2a}$, and $Y^{3a}$ of General Formula (SA), respectively, and preferred ranges thereof are also the same as in General Formula (SA).

Hereinafter, specific examples of the mercaptosilane compound used in the present invention will be given, but the present invention is not limited thereto.

3-Mercaptopropyltrimethoxysilane
3-Mercaptopropyltriethoxysilane
3-Mercaptopropylmethyldimethoxysilane
(Mercaptomethyl)methyldiethoxysilane
(Mercaptomethyl)methyldimethoxysilane
(Mercaptomethyl)dimethylethoxysilane
11-Mercaptoundecyltrimethoxysilane

(Isocyanatosilane compound)

The isocyanatosilane compound (a silane compound having an isocyanato group) is preferably a silane coupling agent having an isocyanato group. The zinc oxide particles surface-treated with the isocyanatosilane compound preferably have an isocyanato group derived from the isocyanatosilane compound.

The isocyanato compound preferably includes at least one compound represented by General Formula (SI).

General Formula (SI) 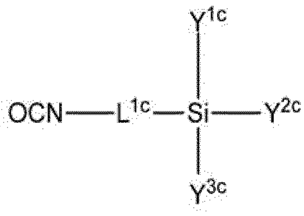

$L^{1c}$, $Y^{1c}$, $Y^{2c}$, and $Y^{3c}$ have the same definition as $L^{1a}$, $Y^{1a}$, $Y^{2a}$, and $Y^{3a}$ of General Formula (SA), respectively, and preferred ranges thereof are also the same as in General Formula (SA).

In addition, in the present invention, it is also preferable to use, as the isocyanatosilane compound, a condensate of the above-described compound represented by General Formula (SI) and a compound in which the isocyanato group of General Formula (SI) is protected by a substituent. The substituent can be introduced by, for example, an alcohol compound, a phenol compound, an aromatic amine, a lactam, or an oxime. Examples of such an alcohol compound include an alkyl alcohol (preferably having 1 to 12 carbon atoms and more preferably 1 to 8 carbon atoms). In addition, examples of the phenol compound include a phenol and a cresol. In addition, examples of the lactam include an ε-caprolactam.

The "compound in which the isocyanato group of General Formula (SI) is protected by a substituent" is a compound in which -NCO of General Formula (SI) is substituted with-NHC(=O)$OR^4$. $R^4$ represents a substituent, examples of which include an alkyl group (preferably having 1 to 12 carbon atoms and more preferably 1 to 8 carbon atoms).

Hereinafter, specific examples of the isocyanatosilane compound used in the present invention will be given, but the present invention is not limited thereto.

3-Isocyanatopropyltrimethoxysilane
3-Isocyanatopropyltriethoxysilane
Isocyanatomethyltrimethoxysilane
(the following are isocyanatosilane compounds protected by condensation and a substituent)
Tris(3-trimethoxysilylpropyl)isocyanurate
(3-Triethoxysilylpropyl)-t-butylcarbamate
Triethoxysilylpropylethylcarbamate

-Thiocyanatosilane compound-

The thiocyanatosilane compound (silane compound having a thiocyanato group) is preferably a silane coupling agent having a thiocyanato group. The zinc oxide particles surface-treated with the thiocyanatosilane compound preferably have an isocyanato group derived from the thiocyanatosilane compound.

The thiocyanatosilane compound preferably includes at least one compound represented by General Formula (ST).

General Formula (ST) 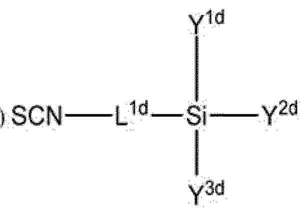

$L^{1d}$, $Y^{1d}$, $Y^{2d}$, and $Y^{3d}$ have the same definition as $L^{1a}$, $Y^{1a}$, $Y^{2a}$, and $Y^{3a}$ of General Formula (SA), respectively, and preferred ranges thereof are also the same as in General Formula (SA).

Hereinafter, specific examples of the thiocyanatosilane compound used in the present invention will be given, but the present invention is not limited thereto.

3-Thiocyanatopropyltrimethoxysilane
3-Thiocyanatopropyltriethoxysilane
Thiocyanatomethyltrimethoxysilane

(Aluminum alkoxide compound)

It is preferable that the aluminum alkoxide compound includes at least one kind of an acetonato structure or an acetato structure.

The aluminum alkoxide compound preferably includes at least one compound represented by General Formula (1).

General Formula (1): $R^{1a}_{m1}\text{-Al-}(OR^{2a})_{3-m1}$

$R^{1a}$ represents a hydrogen atom, an alkyl group, a cycloalkyl group, an acyl group, an aryl group, or an unsaturated aliphatic group.

The alkyl group that can be used as $R^{1a}$ includes a linear alkyl group, a branched alkyl group, and an aralkyl group. The number of carbon atoms in the alkyl group is preferably 1 to 20, more preferably 1 to 15, still more preferably 1 to 10, and particularly preferably 1 to 8, and in a case of an aralkyl group, the number of carbon atoms in the alkyl group is preferably 7 to 30. Preferred specific examples of the alkyl group include methyl, ethyl, propyl, isopropyl, butyl, sec-butyl, isobutyl, tert-butyl, pentyl, hexyl, heptyl, octyl, decyl, tridecyl, octadecyl, benzyl, and phenethyl.

It is also preferable that the alkyl group that can be used as $R^{1a}$ has an oxirane ring. The number of ring members in the cycloalkyl group (cycloalkyl group having a structure in which an oxirane ring is condensed) in the epoxycycloalkyl group that can be used as $R^{1a}$ is preferably 4 to 8, more preferably 5 or 6, and still more preferably 6 (that is, an epoxycyclohexyl group).

In addition, the alkyl group that can be used as $R^{1a}$ preferably has a group selected from an amino group, an isocyanato group, a mercapto group, an ethylenic unsaturated group, and an acid anhydride group.

The cycloalkyl group that can be used as $R^{1a}$ preferably has 3 to 20 carbon atoms, more preferably 3 to 15 carbon atoms, still more preferably 3 to 10 carbon atoms, and particularly preferably 3 to 8 carbon atoms. Preferred specific examples of the cycloalkyl group include cyclopropyl, cyclopentyl, and cyclohexyl.

The acyl group that can be used as $R^{1a}$ preferably has 2 to 40 carbon atoms, more preferably 2 to 30 carbon atoms, still more preferably 2 to 20 carbon atoms, and particularly preferably 2 to 18 carbon atoms.

The aryl group that can be used as $R^{1a}$ preferably has 6 to 20 carbon atoms, more preferably 6 to 15 carbon atoms, still more preferably 6 to 12 carbon atoms, and particularly preferably 6 to 10 carbon atoms. Preferred specific examples of the aryl group include phenyl and naphthyl, and phenyl is still more preferable.

The unsaturated aliphatic group that can be used as $R^{1a}$ preferably has 1 to 5 carbon-carbon unsaturated bonds, more preferably 1 to 3 carbon-carbon unsaturated bonds, still more preferably 1 or 2 carbon-carbon unsaturated bonds, and particularly preferably 1 carbon-carbon unsaturated bond. The unsaturated aliphatic group may contain a heteroatom, and is also preferably a hydrocarbon group. In a case where the unsaturated aliphatic group is a hydrocarbon group, the number of carbon atoms in the group is preferably 2 to 20, more preferably 2 to 15, still more preferably 2 to 10, even still more preferably 2 to 8, and is also preferably 2 to 5. The unsaturated aliphatic group is more preferably an alkenyl group or an alkynyl group.

$R^{1a}$ is preferably a hydrogen atom, an alkyl group, a cycloalkyl group, or an aryl group, and more preferably an alkyl group or a cycloalkyl group.

In a case where the compound of General Formula (1) has two or more $R^{1a}$'s, the two $R^{1a}$'s may be linked to each other to form a ring.

$R^{2a}$ represents a hydrogen atom, an alkyl group, a cycloalkyl group, an acyl group, an alkenyl group, an aryl group, a phosphonate group (phosphonic acid group), or $-SO_2R^{S1}$. $R^{S1}$ represents a substituent.

The alkyl group, cycloalkyl group, acyl group, and aryl group that can be used as $R^{2a}$ have the same definition as the alkyl group, cycloalkyl group, acyl group, and aryl group that can be used as $R^{1a}$, respectively, and a preferred form of each group is also the same as in $R^{1a}$. In addition, the alkyl group that can be used as $R^{2a}$ preferably has an amino group as a substituent.

The alkenyl group that can be used as $R^{2a}$ includes a linear alkenyl group and a branched alkenyl group. The number of carbon atoms in the alkenyl group is preferably 2 to 18, more preferably 2 to 7, and still more preferably 2 to 5. Preferred specific examples of the alkenyl group include vinyl, allyl, butenyl, pentenyl, and hexenyl. The alkenyl group is preferably a substituted alkenyl group.

The phosphonate group that can be used as $R^{2a}$ is a group represented by $-P(=O)(-OR^{P1})OR^{P2}$. $R^{P1}$ and $R^{P2}$ represent a hydrogen atom or a substituent, and the substituent is preferably an alkyl group or a phosphonate group. The alkyl group that can be used as $R^{P1}$ and $R^{P2}$ has the same definition as the alkyl group that can be used as $R^{1a}$ described above, and a preferred form of the alkyl group is also the same as in $R^{1a}$. The phosphonate group that can be used as $R^{P1}$ and $R^{P2}$ has the same definition as the phosphonate group that can be used as $R^{2a}$, and a preferred form thereof is also the same as in $R^{2a}$. In a case where $R^{P1}$ or $R^{P2}$ is a phosphonate group, the $R^{P1}$ and $R^{P2}$ constituting the phosphonate group are each preferably an alkyl group.

As to the phosphonate group that can be used as $R^{2a}$, it is preferable that both $R^{P1}$ and $R^{P2}$ are alkyl groups, or $R^{P1}$ is a hydrogen atom and $R^{P2}$ is a phosphonate group.

Since the phosphonate group is tautomeric with a phosphite group (phosphorous acid group), the phosphonate group in the present invention means to include the phosphite group.

In $-SO_2R^{S1}$ that can be used as $R^{2a}$, the substituent $R^{S1}$ is preferably an alkyl group or an aryl group. Preferred forms of the alkyl group and aryl group that can be used as $R^{S1}$ include the above-described preferred forms of the alkyl group and aryl group that can be used as $R^{1a}$, respectively. Among these, phenyl having an alkyl group as a substituent is preferable for $R^{S1}$. A preferred form of the alkyl group is the same as the above-described preferred form of the alkyl group that can be used as $R^{1a}$.

In a case where the compound represented by General Formula (1) has two or more $R^{2a}$'s, the two $R^{2a}$'s may be linked to each other to form a ring.

m1 is an integer of 0 to 2.

In General Formula (1), it is preferable that at least one of $OR^{2a}$'s has an acetonato structure. The acetonato structure means a structure that one hydrogen ion is removed from acetone or a compound having a structure in which acetone has a substituent, and then the resultant is coordinated to Al. A coordinating atom coordinated to the Al is usually an oxygen atom. The acetonato structure is preferably a structure in which an acetylacetone structure ("$CH_3$-C(=O)-$CH_2$-C(=O)-$CH_3$") is taken as a basic structure, one hydrogen ion is removed from the structure, and the structure is coordinated to Al through an oxygen atom as a coordinating atom (that is, an acetylacetonato structure). The phrase "an acetylacetone structure is taken as a basic structure" means to include a structure in which a hydrogen atom of the acetylacetone structure is substituted with a substituent, in addition to the above-described acetylacetone structure. Examples of the form in which $OR^{2a}$ has an acetonato structure include compounds SL-2 and SL-3 described later.

In General Formula (1), it is preferable that at least one of $OR^{2a}$'s has an acetato structure. In the present invention, the acetato structure means a structure that one hydrogen ion is removed from acetic acid or an acetic acid ester, or a compound having a structure in which the acetic acid or acetic acid ester has a substituent (including a form in which the methyl group of acetic acid has an alkyl group as a substituent), and then the resultant is coordinated to Al. A coordinating atom coordinated to the Al is usually an oxygen atom. The acetato structure is preferably a structure in which an alkylacetoacetato structure ("$CH_3$-C(=O)-$CH_2$-C(=O)-O-$R_{alk}$" ($R_{alk}$ represents an alkyl group (preferably an alkyl group having 1 to 20 carbon atoms, may be an alkyl group having 1 to 10 carbon atoms or an alkyl group having 1 to 4 carbon atoms))) is taken as a basic structure, one hydrogen ion is removed from the structure, and the structure is coordinated to Al through an oxygen atom as a coordinating atom (that is, an alkylacetoacetato structure). The phrase "an alkylacetoacetato structure is taken as a basic structure" means to include a structure in which a hydrogen atom of the alkylacetoacetato structure is substituted with a substituent, in addition to the above-described alkylacetoacetato structure. Examples of the form in which $OR^{2a}$ has an acetato structure include compounds SL-3, SL-4, and SL-5 described later.

The group that can be used as $R^{1a}$ or $R^{2a}$ may have an anionic group having a counter cation (salt-type substituent) as a substituent. The anionic group means a group capable of forming an anion. Examples of the anionic group having a counter cation include a carboxylic acid ion group having an ammonium ion as a counter cation. In this case, the counter cation may be present in the compound represented by General Formula (1) such that a charge of the entire compound is zero. This also applies to a compound represented by General Formula (2) and a compound represented by General Formula (3), which will be described later.

Hereinafter, specific examples of the aluminum alkoxide compound used in the present invention will be given, but the present invention is not limited thereto.

Aluminum triethylate
Aluminum triisopropylate
Aluminum tri-sec-butyrate
Aluminum tris(ethylacetoacetate)
Ethyl acetoacetate aluminum diisopropylate
Aluminum monoacetylacetonate bis(ethylacetoacetate)
Aluminum tris(acetylacetonate)
Diisopropoxy aluminum-9-octadecenylacetoacetate
Aluminum diisopropoxy monoethyl acetoacetate
Aluminum trisacetylacetonate
Mono sec-butoxyaluminum diisopropylate
Diethylacetoacetate aluminum isopropylate
Aluminum bisethylacetoacetate monoacetylacetonate
Aluminum octadecylacetoacetate diisopropylate

(Zirconium alkoxide compound)

The zirconium alkoxide compound preferably includes at least one kind of an acetonato structure, an acetato structure, or a lactato structure, and more preferably includes at least one kind of an acetonato structure or an acetato structure.

The zirconium alkoxide compound preferably includes at least one compound represented by General Formula (2).

General Formula (2): $R^{1b}_{m2}$-Zr-$(OR^{2b})_{4-m2}$

$R^{1b}$ represents a hydrogen atom, an alkyl group, a cycloalkyl group, an acyl group, an aryl group, or an unsaturated aliphatic group.

As the alkyl group, the cycloalkyl group, the acyl group, the aryl group, and the unsaturated aliphatic group, for example, an alkyl group, a cycloalkyl group, an acyl group, an aryl group, and an unsaturated aliphatic group that can be used as $R^{1a}$ of General Formula (1) can be adopted.

$R^{2b}$ represents a hydrogen atom, an alkyl group, a cycloalkyl group, an acyl group, an alkenyl group, an aryl group, a phosphonate group, or $-SO_2R^{S2}$. $R^{S2}$ represents a substituent.

As the alkyl group, the cycloalkyl group, the acyl group, the alkenyl group, the aryl group, and the phosphonate group, for example, an alkyl group, a cycloalkyl group, an acyl group, an alkenyl group, an aryl group, and a phosphonate group that can be used as $R^{2a}$ of General Formula (1) can be adopted. In addition, as the substituent that can be used as $R^{S2}$, for example, a substituent that can be used as $R^{S1}$ of General Formula (1) can be adopted.

m2 is an integer of 0 to 3.

In General Formula (2), it is preferable that at least one of $OR^{2b}$'s has an acetonato structure. The acetonato structure has the same definition as the acetonato structure described by General Formula (1). Examples of the form in which $OR^{2b}$ has an acetonato structure include compounds SZ-3 and SZ-6 described later.

In addition, in General Formula (2), it is preferable that at least one of $OR^{2b}$'s has an acetato structure. The acetato structure has the same definition as the acetato structure described by General Formula (1). Examples of the form in which $OR^{2b}$ has an acetato structure include a compound SZ-7 described later.

In addition, in General Formula (2), it is preferable that at least one of $OR^{2b}$'s has a lactato structure. The lactato structure means a structure in which a lactic acid ion (lactate) is taken as a basic structure, and one hydrogen ion is removed from the basic structure, and the structure is coordinated to Zr. The phrase "a lactic acid ion is taken as a basic structure" means to include, in addition to the lactic acid ion, a structure in which a hydrogen atom of the lactic acid ion is substituted with a substituent. A coordinating atom coordinated to the Zr is usually an oxygen atom. Examples of the form in which $OR^{2b}$ has a lactato structure include a compound SZ-4 described later.

Hereinafter, specific examples of the zirconium alkoxide compound used in the present invention will be given, but the present invention is not limited thereto.

Tetrapropoxyzirconium (also known as zirconium tetra-n-propoxide)
Tetrabutoxyzirconium (also known as zirconium tetra-n-butoxide)
Zirconium tetraacetylacetonate
Zirconium tributoxy monoacetylacetonate
Zirconium dibutoxy bis(acetyl acetonate)
Zirconium dibutoxy bis(ethyl acetoacetate)
Zirconium tributoxyethylacetoacetate
Zirconium monobutoxyacetylacetonate bis(ethyl acetoacetate)
Zirconium tributoxy monostearate (also known as zirconium stearate tri-n-butoxide)
Zirconium stearate
Zirconium lactate ammonium salt
Zirconium monoacetylacetonate

(Titanium alkoxide compound)

It is preferable that the titanium alkoxide compound includes at least one atom of N, P, or S. In addition, it is also preferable that the titanium alkoxide compound has an acetato structure.

The titanium alkoxide compound preferably includes at least one compound represented by General Formula (3).

General Formula (3): $R^{1c}_{m3}$-Ti-$(OR^{2c})_{4-m3}$

$R^{1c}$ represents a hydrogen atom, an alkyl group, a cycloalkyl group, an acyl group, an aryl group, or an unsaturated aliphatic group.

As the alkyl group, the cycloalkyl group, the acyl group, the aryl group, and the unsaturated aliphatic group, for example, an alkyl group, a cycloalkyl group, an acyl group, an aryl group, and an unsaturated aliphatic group that can be used as $R^{1a}$ of General Formula (1) can be adopted.

$R^{2c}$ represents a hydrogen atom, an alkyl group, a cycloalkyl group, an acyl group, an alkenyl group, an aryl group, a phosphonate group, or $-SO_2R^{S3}$. $R^{S3}$ represents a substituent.

As the alkyl group, the cycloalkyl group, the acyl group, the alkenyl group, the aryl group, and the phosphonate group, for example, an alkyl group, a cycloalkyl group, an acyl group, an alkenyl group, an aryl group, and a phosphonate group that can be used as $R^{2a}$ of General Formula (1) can be adopted. In addition, as the substituent that can be used as $R^{S3}$, for example, a substituent that can be used as $R^{S1}$ of General Formula (1) can be adopted.

m3 is an integer of 0 to 3.

The compound represented by General Formula (3) preferably contains at least one atom of N, P, or S. In a case where the compound represented by General Formula (3) has N, it is preferable to have the N as an amino group.

In a case where the compound represented by General Formula (3) has P, it is preferable to have the P as a phosphate group (phosphoric acid group) or a phosphonate group (phosphonic acid group).

In a case where the compound represented by General Formula (3) has S, it is preferable to have the S as a sulfonyl group ($-SO_2-$).

In addition, it is also preferable that the compound represented by General Formula (3) has an acyl group as $R^{2c}$, that is, has the above-described acetato structure as $OR^{2c}$.

Hereinafter, specific examples of the zirconium alkoxide compound used in the present invention will be given, but the present invention is not limited thereto.

Isopropyltriisostearoyl titanate
Isopropyltridodecylbenzenesulfonyl titanate
Isopropyltrioctanoyl titanate
Isopropyltri(dioctylphosphite)titanate
Isopropyltri s(dioctylpyrophosphate)titanate
Isopropyltri(dioctylsulfate)titanate
Isopropyltricumylphenyl titanate
Isopropyltri(N-aminoethyl-aminoethyl)titanate
Isopropyldimethacryl isostearoyl titanate
Isopropylisostearoyl diacryl titanate
Isobutyltrimethyl titanate
Diisostearoylethylene titanate
Diisopropyl bis(dioctylpyrophosphate)titanate
Dioctyl bis(ditridecylphosphate)titanate
Dicumyl phenyl oxyacetate titanate
Bis(dioctylpyrophosphate)oxyacetate titanate
Bis(dioctylpyrophosphate)ethylene titanate
Tetraisopropyl titanate
Tetrabutyl titanate
Tetraoctyl titanate
Tetrastearyl titanate
Tetraisopropyl bis(dioctylphosphite)titanate
Tetraoctyl bis(di-tridecylphosphite)titanate
Tetra(2,2-diallyloxymethyl-1 -butyl)bis(di-tridecyl)phosphite titanate
Butyl titanate dimer
Titanium tetraacetylacetonate
Titanium ethyl acetoacetate
Titanium octylene glycolate
Titanium di-2-ethylhexoxybis(2-ethyl-3-hydroxyhexoxide)

From the viewpoint of acoustic velocity, adhesiveness, and ozone water resistance of the acoustic lens, the surface treatment agent used in the present invention is preferably an aminosilane compound, a mercaptosilane compound, an isocyanatosilane compound, an aluminum alkoxide compound, a zirconium alkoxide compound, or a titanium alkoxide compound; more preferably an aminosilane compound, a mercaptosilane compound, an isocyanatosilane compound, an aluminum alkoxide compound, or a zirconium alkoxide compound; still more preferably an aminosilane

compound, an isocyanatosilane compound, an aluminum alkoxide compound, or a zirconium alkoxide compound; even more preferably an isocyanatosilane compound, an aluminum alkoxide compound, or a zirconium alkoxide compound; even still more preferably an aluminum alkoxide compound or a zirconium alkoxide compound; and particularly preferably an aluminum alkoxide compound.

A mass ratio of the zinc oxide particles and the surface treatment agent in the component (D) is not particularly limited, and for example, with respect to 100 parts by mass of the zinc oxide particles, the content of the surface treatment agent can be 1 to 100 parts by mass, preferably 10 to 80 parts by mass, more preferably 10 to 60 parts by mass, still more preferably 20 to 50 parts by mass, and particularly preferably 20 to 40 parts by mass, from the viewpoint of acoustic velocity, adhesiveness, and ozone water resistance of the acoustic lens.

The mass ratio of the zinc oxide particles and the surface treatment agent in the component (D) is the same as a mass ratio of a used amount of the zinc oxide particles and a used amount of the surface treatment agent in the surface treatment. The mass ratio of the zinc oxide particles and the surface treatment agent in the component (D) can be calculated from the mass of the zinc oxide particles and the mass of the component (D) with a thermogravimetric analysis (TGA) or the like by heating the component (D) to 500°C or higher to remove an organic component to obtain an inorganic component (zinc oxide particles).

A surface treatment agent other than the above-described surface treatment agent may be used as long as the effects of the present invention are not impaired.

The above-described surface treatment can be performed by a common method.

As for the component (D), it is not necessary that the entire surface of the zinc oxide particles is treated with the surface treatment agent. For example, it is preferable that 50% or more of 100% surface area of the zinc oxide particles is surface-treated, more preferable that 70% or more thereof is surface-treated, and still more preferable that 90% or more thereof is surface-treated.

The component (D) may be used alone or in combination of two or more thereof.

<Other components>

In addition to the components (A) to (D), at least one of a catalyst for an addition polymerization reaction, a curing retarder, a solvent, a dispersant, a pigment, a dye, an antistatic agent, an antioxidant, a flame retardant, a thermal conductivity improver, or the like can be appropriately formulated in the composition for an acoustic lens according to the embodiment of the present invention.

- Catalyst -

Examples of the catalyst include platinum or a platinum-containing compound (hereinafter, also simply referred to as a platinum compound). An ordinary platinum or platinum compound can be used as the platinum or platinum compound.

Specific examples thereof include platinum black or platinum supported on an inorganic compound or carbon black, chloroplatinic acid or an alcohol solution of chloroplatinic acid, a complex salt of chloroplatinic acid and olefin, and a complex salt of chloroplatinic acid and vinyl siloxane. The catalyst may be used alone or in combination of two or more thereof.

The catalyst is effective in a hydrosilylation reaction in which the Si-H group is added to the vinyl group. By the progress of the hydrosilylation reaction (addition curing reaction), it is possible to form a highly three-dimensional network structure formed of the components (A) to (C). A molar ratio of the Si-H group and the vinyl group in the hydrosilylation reaction can be determined based on a stoichiometric ratio, but is not limited thereto.

Here, the catalyst may be contained in the composition for an acoustic lens according to the embodiment of the present invention, or may be brought into contact with the composition for an acoustic lens without being contained in the composition for an acoustic lens.

Examples of commercially available platinum catalysts include platinum compounds (trade name: PLATINUM CYCLOVINYLMETHYLSILOXANE COMPLEX IN CYCLIC METHYLVINYLSILOXANES (SIP6832.2), Pt concentration: 2% by mass, and trade name: PLATINUM DIVINYLTETRAMETHYLDISILOXANE COMPLEX IN VINYL-TERMINATED POLYDIMETHYLSILOXANE (SIP6830.3), Pt concentration: 3% by mass; both manufactured by Gelest, Inc.).

In a case where the catalyst is contained in the composition for an acoustic lens according to the embodiment of the present invention, the content of the catalyst is not particularly limited. From the viewpoint of reactivity, the content of the catalyst is preferably 0.00001 to 0.05 parts by mass, more preferably 0.00001 to 0.01 parts by mass, still more preferably 0.00002 to 0.01 parts by mass, and particularly preferably 0.00005 to 0.005 parts by mass with respect to the total of 100 parts by mass of the components (A) to (D).

In addition, the curing temperature can be adjusted by selecting an appropriate platinum catalyst. For example, platinum-vinyl disiloxane is used for room temperature curing (RTV) at 50°C or lower, and platinum-cyclic vinyl siloxane

is used for high temperature curing (HTV) at 130°C or higher.

- Curing retarder -

In the present invention, a curing retarder for the curing reaction can be appropriately used. The curing retarder is used for the purpose of delaying the above-described addition curing reaction, and examples thereof include a low molecular weight vinyl methylsiloxane homopolymer (trade name: VMS-005, manufactured by Gelest, Inc.).
A curing rate, that is, a working time can be adjusted depending on the content of the curing retarder.

[Viscosity of composition for acoustic lens before curing]

A viscosity of the composition for an acoustic lens before the curing reaction is preferably low from the viewpoint of uniformly dispersing the components (A) to (D). The viscosity of the composition for an acoustic lens is measured before adding the catalyst for initiating the curing reaction, from the viewpoint of measuring the viscosity before curing. Specifically, the viscosity of the composition for an acoustic lens can be measured by the method described in WO2017/130890A.
The above-described viscosity (23°C) is preferably 5,000 Pa s or less, more preferably 1,000 Pa s or less, and particularly preferably 200 Pa s or less. The practical lower limit thereof is 10 Pa·s or more.

<Method for manufacturing composition for acoustic wave lens, acoustic lens, and acoustic wave probe>

The composition for an acoustic lens according to the embodiment of the present invention can be prepared by a conventional method.
The composition for an acoustic lens according to the embodiment of the present invention can be obtained, for example, by kneading the components constituting the composition for an acoustic lens using a kneader, a pressurized kneader, a Banbury mixer (continuous kneader), a two-roll kneading device, or the like. The mixing order of each component is not particularly limited.
From the viewpoint of obtaining a uniform composition, it is preferable to first prepare a polysiloxane mixture in which the component (D) is dispersed in the components (A) to (C). Thereafter, a composition for an acoustic lens can be produced by adding the catalyst to the polysiloxane mixture in which the component (D) is dispersed, followed by defoaming under reduced pressure.
The conditions for kneading the polyorganosiloxane mixture in which the component (D) is dispersed are not particularly limited as long as the component (D) is dispersed. For example, it is preferable to knead the polyorganosiloxane mixture at 10°C to 50°C for 1 to 72 hours.
A silicone resin can be obtained by curing the composition for an acoustic lens according to the embodiment of the present invention thus obtained. Specifically, for example, a silicone resin can be obtained by heat-curing the composition for an acoustic lens at 20°C to 200°C for 5 to 500 minutes. A shape of the above-described silicone resin is not particularly limited. For example, the silicone resin may be formed into a preferred shape as an acoustic lens by a mold during the curing, or may be used as a desired acoustic lens by obtaining a sheet-like silicone resin and cutting the resin.
The composition for an acoustic lens according to the embodiment of the present invention is useful for medical members, and can be preferably used, for example, in an acoustic wave probe and an acoustic wave measurement apparatus. The acoustic wave measurement apparatus according to the embodiment of the present invention is not limited to an ultrasound diagnostic apparatus or a photoacoustic wave measurement apparatus, but refers to a device that receives an acoustic wave reflected or generated by an object and displays the received acoustic wave as an image or a signal intensity.
In particular, the composition for an acoustic lens according to the embodiment of the present invention can be suitably used as a material for an acoustic lens of a probe for an ultrasound diagnostic apparatus, a material for an acoustic lens in a photoacoustic wave measurement apparatus or an ultrasonic endoscope, a material for an acoustic lens in an ultrasound probe equipped with a capacitive micromachined ultrasonic transducer (cMUT) as an ultrasonic transducer array, or the like.
Specifically, the acoustic lens according to the embodiment of the present invention is preferably applied to an acoustic wave measurement apparatus such as the ultrasound diagnostic apparatus described in JP2003-169802A and the like, or the photoacoustic wave measurement apparatus described in JP2013-202050A, JP2013-188465A, and the like.
The acoustic wave probe according to the embodiment of the present invention can be manufactured by a conventional method, except that an acoustic lens is formed of the composition for an acoustic lens according to the embodiment of the present invention.

<<Acoustic wave probe>>

The configuration of the acoustic wave probe according to the embodiment of the present invention will be described in more detail below based on the configuration of the ultrasound probe in the ultrasound diagnostic apparatus described in Fig. 1. The ultrasound probe is a probe which particularly uses an ultrasonic wave as an acoustic wave in an acoustic wave probe. Therefore, a basic structure of the ultrasound probe can be applied to the acoustic wave probe as it is.

- Ultrasound probe -

An ultrasound probe 10 is a main component of the ultrasound diagnostic apparatus and has a function of generating an ultrasonic wave and transmitting and receiving an ultrasonic beam. As shown in Fig. 1, a configuration of the ultrasound probe 10 is provided in the order of an acoustic lens 1, an acoustic matching layer 2, a piezoelectric element layer 3, and a backing material 4 from a distal end portion (surface coming into contact with a living body which is a test object). In recent years, an ultrasound probe having a laminated structure in which an ultrasonic transducer (piezoelectric element) for transmission and an ultrasonic transducer (piezoelectric element) for reception are formed of materials different from each other has been proposed in order to receive high-order harmonics.

<Piezoelectric element layer>

The piezoelectric element layer 3 is a portion which generates an ultrasonic wave and in which an electrode is attached to both sides of a piezoelectric element. In a case where voltage is applied to the electrode, the piezoelectric element layer 3 generates an ultrasonic wave through repeated contraction and expansion of the piezoelectric element and through vibration.

A so-called ceramics inorganic piezoelectric body obtained by a polarization treatment of quartz crystals, single crystals such as $LiNbO_3$, $LiTaO_3$, and $KNbO_3$, thin films of ZnO and AlN, $Pb(Zr,Ti)O_3$-based sintered body, and the like is widely used as the material constituting a piezoelectric element. In general, piezoelectric ceramics such as lead zirconate titanate (PZT) with good conversion efficiency are used.

In addition, sensitivity having a wider band width is required for a piezoelectric element detecting a reception wave on a high frequency side. For this reason, an organic piezoelectric body has been used in which an organic polymer material such as polyvinylidene fluoride (PVDF) is used as the piezoelectric element being suitable for a high frequency or a wide band.

Furthermore, cMUT using micro electro mechanical systems (MEMS) technology in which an array structure, which shows excellent short pulse characteristics, excellent wideband characteristics, and excellent mass productivity and has less characteristic variations, is obtained is described in JP2011-071842A or the like.

In the present invention, it is possible to preferably use any piezoelectric element material.

<Backing material>

The backing material 4 is provided on a rear surface of the piezoelectric element layer 3 and contributes to the improvement in distance resolution in an ultrasound diagnostic image by shortening the pulse width of an ultrasonic wave through the suppression of excess vibration.

<Acoustic matching layer>

The acoustic matching layer 2 is provided in order to reduce the difference in acoustic impedance between the piezoelectric element layer 3 and a test object and to efficiently transmit and receive an ultrasonic wave.

<Acoustic lens>

The acoustic lens 1 is provided to focus an ultrasonic wave in a slice direction by utilizing refraction to improve the resolution. In addition, it is necessary for the acoustic lens to achieve matching of an ultrasonic wave with acoustic impedance (1.4 to 1.7 Mrayl in a case of a human body) of a living body which is a test object after being closely attached to the living body.

That is, sensitivity of transmission and reception of an ultrasonic wave is improved using a material of which the acoustic velocity is sufficiently lower than that of a human body, and the acoustic impedance is close to a value of the skin of a human body, as the material of the acoustic lens 1.

The composition for an acoustic lens according to the embodiment of the present invention can be preferably

used as an acoustic lens material.

The operation of the ultrasound probe 10 having such a configuration will be described. The piezoelectric element layer 3 is resonated after applying a voltage to the electrodes provided on both sides of the piezoelectric element layer 3, and an ultrasonic signal is transmitted to a test object from the acoustic lens 1. During reception of the ultrasonic signal, the piezoelectric element layer 3 is vibrated using the signal (echo signal) reflected from the test object and this vibration is electrically converted into a signal to obtain an image.

In particular, it is possible to confirm for the acoustic lens obtained from the composition for an acoustic lens according to the embodiment of the present invention to have a significant sensitivity improving effect at a transmission frequency of an ultrasonic wave of about 10 MHz or higher as a general medical ultrasonic transducer. A particularly significant sensitivity improving effect can be expected particularly at a transmission frequency of an ultrasonic wave of 15 MHz or higher.

The acoustic lens obtained from the composition for an acoustic lens according to the embodiment of the present invention can reduce the acoustic velocity to, for example, less than 900 m/s, and with an ultrasonic frequency of 10 MHz or more and less than 50 MHz, it is possible to obtain sufficiently accurate information about with regard to living tissues at a depth of 0.1 to 20 mm from a body surface.

Hereinafter, a device in which the acoustic lens obtained from the composition for an acoustic lens according to the embodiment of the present invention exerts a particular function with respect to the problems of the related art will be described in detail.

The composition for an acoustic lens according to the embodiment of the present invention also exhibits excellent effects on devices other than those described below.

- Ultrasound probe equipped with capacitive micromachined ultrasonic transducer (cMUT) -

In a case where the cMUT device described in JP2006-157320A, JP2011-71842A, or the like is used in an ultrasonic transducer array, its sensitivity is generally lower than that of a transducer using general piezoelectric ceramics (PZT).

However, by using the acoustic lens obtained from the composition for an acoustic lens according to the embodiment of the present invention, it is possible to compensate for the lack of sensitivity of cMUT. As a result, it is possible to bring the sensitivity of the cMUT closer to the performance of a transducer of the related art.

Since the cMUT device is produced by MEMS technology, it is possible to provide the market with an ultrasound probe having higher mass productivity and lower cost than a piezoelectric ceramic probe.

- Photoacoustic wave measurement apparatus using photoacoustic imaging -

Photoacoustic imaging (PAI) described in JP2013-158435A or the like displays an image or a signal intensity of an ultrasonic wave generated in a case where the inside of a human body is irradiated with light (an electromagnetic wave), and the human tissue adiabatically expands due to the irradiated light.

- Ultrasonic endoscope -

Since the signal line cable of the ultrasonic endoscope described in JP2008-311700A or the like is longer than that of a transducer for the body surface due to its structure, it is a problem to improve the sensitivity of the transducer due to the cable loss of an ultrasonic wave. In addition, it is said that there is no effective method for improving sensitivity for this problem for the following reasons.

First, in a case where it is an ultrasound diagnostic apparatus for the body surface, an amplifier circuit, an AD conversion IC, or the like can be installed at the tip of a transducer. On the other hand, since an ultrasonic endoscope is used by insertion thereof into the body, an installation space of a transducer is narrow, and it is difficult to install an amplifier circuit, an AD conversion IC, or the like at the tip of the transducer.

Secondly, a piezoelectric single crystal used in a transducer in an ultrasound diagnostic apparatus for the body surface is difficult to apply to a transducer with a transmission frequency of an ultrasonic wave of 10 to 15 MHz or higher due to its physical characteristics and process suitability. Meanwhile, since an ultrasonic wave for an endoscope is generally a probe having a transmission frequency of an ultrasonic wave of 10 to 15 MHz or higher, it is difficult to improve the sensitivity by using a piezoelectric single crystal material.

However, by using the acoustic lens obtained from the composition for an acoustic lens according to the embodiment of the present invention, it is possible to improve the sensitivity of an ultrasonic transducer for an endoscope.

In addition, even in a case where the same transmission frequency of an ultrasonic wave (for example, 15 MHz) is used, it is particularly effective in a case where the acoustic lens obtained from the composition for an acoustic lens according to the embodiment of the present invention is used in an ultrasonic transducer for an endoscope.

Examples

The present invention will be described in more detail based on Examples in which an ultrasonic wave was used as an acoustic wave. The present invention is not limited to Examples below, except as specified in the present invention.

[Preparation Example] Preparation example of surface-treated zinc oxide particles (D-1)

3.0 parts by mass of methylmethoxysilane, 100 parts by mass of methanol, and 10 parts by mass of distilled water were mixed with each other, and then allowed to stand at 23°C for 2 hours to proceed hydrolysis of the methoxy group. 10.0 parts by mass of zinc oxide particles (manufactured by Sakai Chemical Industry Co., Ltd., trade name "FINEX-30", average primary particle diameter: 35 nm) was added to the solution. Using a homogenizer ("AUTO EXCEL HOMOGENIZER ED-7" (trade name), manufactured by Nippon Seiki Co., Ltd.), the mixture was stirred at a rotation speed of 10,000 rpm for 60 minutes while cooling such that the liquid temperature did not exceed 50°C, and a surface treatment was carried out while pulverizing.

The mixture after stirring and pulverizing above was filtered off, and the obtained solid was heated and dried at 100°C for 30 minutes to obtain powdery surface-treated zinc oxide particles (D-1) (component (D)).

Surface-treated zinc oxide particles (D-2) to (D-37) were prepared in the same manner as in the surface-treated zinc oxide particles (D-1), except that, in the preparation of the surface-treated zinc oxide particles (D-1), the raw materials were used with the formulation shown in Table 1. In Comparative Example 8 described later, commercially available surface-treated silica (Q-7) was used as it is.

[Table 1]

| | | D-1 | D-2 | D-3 | D-4 | D-5 | D-6 | D-7 | D-8 | D-9 | D-10 |
|---|---|---|---|---|---|---|---|---|---|---|---|
| Zinc oxide (Q) | Type | Q-1 | Q-1 | Q-1 | Q-1 | Q-1 | Q-1 | Q-1 | Q-1 | Q-1 | Q-1 |
| | Average primary particle diameter [nm] | 35 | 35 | 35 | 35 | 35 | 35 | 35 | 35 | 35 | 35 |
| Surface treatment agent (S) | Type | SC-1 | SC-2 | SC-3 | SC-4 | SC-5 | SC-6 | SA-1 | SA-2 | SM-1 | SM-2 |
| | Number of acetonato structures | - | - | - | - | - | - | - | - | - | - |
| | Number of acetate structures | - | - | - | - | - | - | - | - | - | - |
| | Used amount [part by mass] | 30 | 30 | 30 | 30 | 30 | 30 | 30 | 30 | 30 | 30 |

| | | D-11 | D-12 | D-13 | D-14 | D-15 | D-16 | D-17 | D-18 | D-19 | D-20 |
|---|---|---|---|---|---|---|---|---|---|---|---|
| Zinc oxide (Q) | Type | Q-1 | Q-1 | Q-1 | Q-1 | Q-1 | Q-1 | Q-1 | Q-1 | Q-1 | Q-1 |
| | Average primary particle diameter [nm] | 35 | 35 | 35 | 35 | 35 | 35 | 35 | 35 | 35 | 35 |

(continued)

| | | D-11 | D-12 | D-13 | D-14 | D-15 | D-16 | D-17 | D-18 | D-19 | D-20 |
|---|---|---|---|---|---|---|---|---|---|---|---|
| Surface treatment agent (S) | Type | SI-1 | SI-2 | ST-1 | ST-2 | ST-3 | SL-1 | SL-2 | SL-3 | SL-4 | SL-5 |
| | Number of acetonato structures | - | - | - | - | - | - | 3 | 1 | - | - |
| | Number of acetate structures | - | - | - | - | - | - | - | 2 | 3 | 1 |
| | Used amount [part by mass] | 30 | 30 | 30 | 30 | 30 | 30 | 30 | 30 | 30 | 30 |

| | | D-21 | D-22 | D-23 | D-24 | D-25 | D-26 | D-27 | D-28 | D-29 | D-30 |
|---|---|---|---|---|---|---|---|---|---|---|---|
| Zinc oxide (Q) | Type | Q-1 | Q-1 | Q-1 | Q-1 | Q-1 | Q-1 | Q-1 | Q-2 | Q-3 | Q-4 |
| | Average primary particle diameter [nm] | 35 | 35 | 35 | 35 | 35 | 35 | 35 | 20 | 60 | 200 |
| Surface treatment agent (S) | Type | SZ-1 | SZ-2 | SZ-3 | SZ-4 | SZ-5 | SZ-6 | SZ-7 | SL-4 | SL-4 | SL-4 |
| | Number of acetonato structures | - | - | 4 | - | - | 1 | - | - | - | - |
| | Number of acetate structures | - | - | - | - | - | - | - | 2 | - | - |
| | Used amount [part by mass] | 30 | 30 | 30 | 30 | 30 | 30 | 30 | 30 | 30 | 30 |

| | | D-31 | D-32 | D-33 | D-34 | D-35 | D-36 | D-37 |
|---|---|---|---|---|---|---|---|---|
| Zinc oxide (Q) | Type | Q-5 | Q-1 | Q-1 | Q-1 | Q-1 | Q-1 | Q-6 |
| | Average primary particle diameter [nm] | 250 | 35 | 35 | 35 | 35 | 35 | 350 |
| Surface treatment agent (S) | Type | SL-4 | SL-4 | SL-4 | SS-1 | SS-2 | SS-3 | SL-4 |
| | Number of acetonato structures | - | - | - | - | - | - | - |
| | Number of acetate structures | - | - | - | - | - | - | - |
| | Used amount [part by mass] | 30 | 10 | 50 | 30 | 30 | 30 | 30 |

<Notes of table>

[Zinc oxide particles (Q)] (in Table 1, described as Zinc oxide (Q))

Q-1: untreated zinc oxide particles (manufactured by Sakai Chemical Industry Co., Ltd., trade name "FINEX-30", average particle diameter: 35 nm)
Q-2: untreated zinc oxide particles (manufactured by Sakai Chemical Industry Co., Ltd., trade name "FINEX-50", average particle diameter: 20 nm)

Q-3: untreated zinc oxide particles (manufactured by Sakai Chemical Industry Co., Ltd., trade name "FINEX-25", average particle diameter: 60 nm)
Q-4: untreated zinc oxide particles (manufactured by SUMITOMO OSAKA CEMENT Co., Ltd., trade name "ZnO-S05", average particle diameter: 200 nm)
Q-5: untreated zinc oxide particles (manufactured by SUMITOMO OSAKA CEMENT Co., Ltd., trade name "ZnO-CX", average particle diameter: 250 nm)

-Inorganic particles used in Comparative Examples-

Q-6: zinc oxide particles for heat radiation (manufactured by HAKUSUI TECH., trade name "Ultrafine-particle zinc oxide", average particle diameter: 350 nm)
Q-7: surface-treated silica (trade name "QSG-30" manufactured by Shin-Etsu Chemical Co., Ltd., average primary particle diameter: 30 nm, surface-treated product with methyltrimethoxysilane (MTMS) and hexamethyldisilazane (HMDS))
Q-7 is shown in Table 2-3, but is shown in <Notes of table> of Table 1 for easy comparison with Q-1 to Q-6.

[Surface treatment agent (S)]

<Silane alkoxide compound>

(SC-1):
methyltrimethoxysilane (manufactured by Shin-Etsu Chemical Co., Ltd., trade name "KBM-13")
(SC-2):
n-propyltrimethoxysilane (manufactured by Shin-Etsu Chemical Co., Ltd., trade name "KBM-3033")
(SC-3):
n-octyltriethoxysilane (manufactured by Shin-Etsu Chemical Co., Ltd., trade name "KBM-3083")
(SC-4):
n-decyltrimethoxysilane (manufactured by Shin-Etsu Chemical Co., Ltd., trade name "KBM-3103C")
(SC-5):
n-hexadecyltrimethoxysilane (Tokyo Chemical Industry Co., Ltd.)
(SC-6):
phenyltrimethoxysilane (manufactured by Shin-Etsu Chemical Co., Ltd., trade name "KBM-103")
(SA-1):
3-aminopropyltrimethoxysilane (manufactured by Shin-Etsu Chemical Co., Ltd., trade name "KBM-903")
(SA-2):
N-(2-aminoethyl)-3-aminopropyltrimethoxysilane (manufactured by Shin-Etsu Chemical Co., Ltd., trade name "KBM-603")
(SM-1):
3-mercaptopropyltrimethoxysilane (manufactured by Shin-Etsu Chemical Co., Ltd., trade name "KBM-803")
(SM-2):
11-mercaptoundecyltrimethoxysilane (manufactured by Gelest, Inc., trade name "SIM6480.0")
(SI-1):
3-isocyanatopropyltrimethoxysilane (manufactured by Gelest, Inc., trade name "SII6456.0")
(SI-2):
isocyanatomethyltrimethoxysilane (manufactured by Gelest, Inc., trade name "SII6453.8")

<Titanium alkoxide compound>

(ST-1):
isopropyltriisostearoyl titanate (manufactured by Ajinomoto Fine-Techno Co., Inc., trade name "PLENACT TTS")

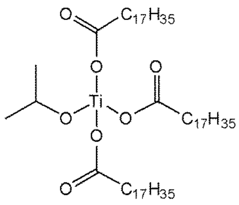

ST-1

(ST-2):
dioctyl bis(ditridecylphosphate)titanate ("PLENACT 46B" manufactured by Ajinomoto Fine-Techno Co., Inc.)

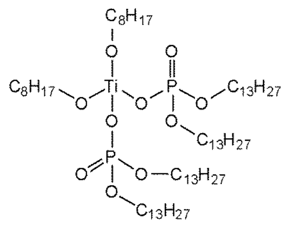

ST-2

(ST-3):
isopropyl tri(N-aminoethyl-aminoethyl)titanate (manufactured by Ajinomoto Fine-Techno Co., Inc., trade name "PLENACT 44")

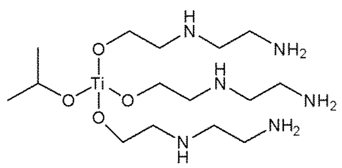

ST-3

<Aluminum alkoxide compound>

(SL-1):
aluminum tri-sec-butyrate (manufactured by Kawaken Fine Chemicals Co., Ltd., trade name "ASBD")

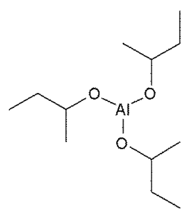

SL-1

(SL-2):
aluminum trisacetylacetonate (manufactured by Matsumoto Fine Chemical Co., Ltd., trade name "ORGATIX AL-3100")

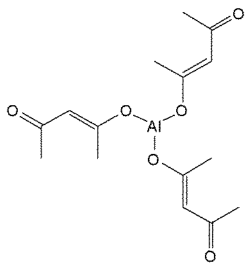

SL-2

(SL-3):
aluminum bisethylacetoacetate monoacetylacetonate (manufactured by Matsumoto Fine Chemical Co., Ltd., trade name "ORGATIX AL-3200")

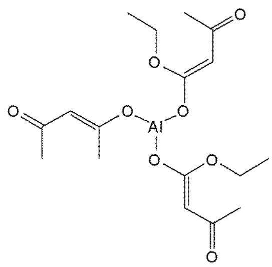

SL-3

(SL-4):
aluminum tris(ethylacetoacetate) (manufactured by Matsumoto Fine Chemical Co., Ltd., trade name "ORGATIX AL-3215")

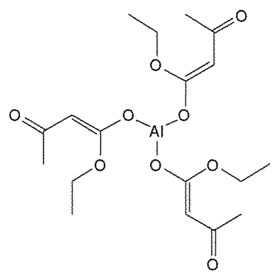

SL-4

(SL-5):
aluminum octadecylacetoacetate diisopropylate (manufactured by Ajinomoto Fine-Techno Co., Inc., trade name "PLENACT AL-M")

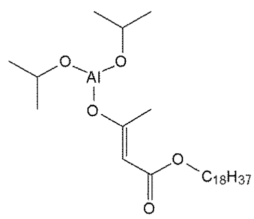

SL-5

<Zirconium alkoxide compound>

(SZ-1):
zirconium tetra-n-propoxide (manufactured by Matsumoto Fine Chemical Co., Ltd., trade name "ORGATIX ZA-45")

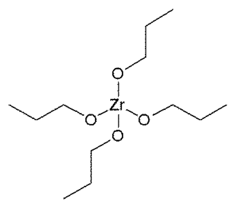

SZ-1

(SZ-2):

zirconium tetra-n-butoxide (manufactured by Matsumoto Fine Chemical Co., Ltd., trade name "ORGATIX ZA-65")

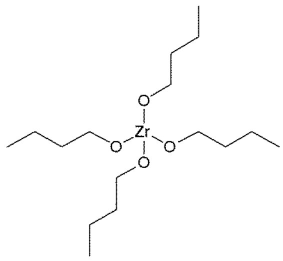

SZ-2

(SZ-3):

zirconium tetraacetylacetonate (manufactured by Matsumoto Fine Chemical Co., Ltd., trade name "ORGATIX ZC-150")

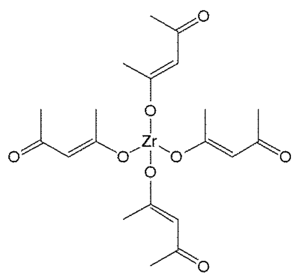

SZ-3

(SZ-4):

zirconium lactate ammonium salt (manufactured by Matsumoto Fine Chemical Co., Ltd., trade name "ORGATIX ZC-300")

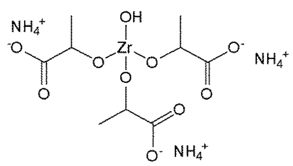

SZ-4

(SZ-5):

zirconium stearate tri-n-butoxide (manufactured by Matsumoto Fine Chemical Co., Ltd., trade name "ORGATIX ZC-320")

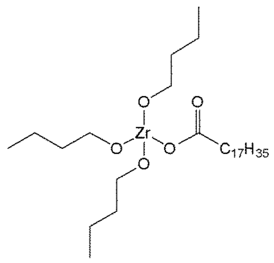

SZ-5

(SZ-6):
zirconium tributoxy monoacetylacetonate (manufactured by Matsumoto Fine Chemical Co., Ltd., trade name "ORGATIX ZC-540")

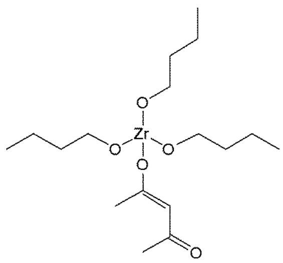

SZ-6

(SZ-7):
zirconium dibutoxy bis(ethyl acetoacetate) (manufactured by Matsumoto Fine Chemical Co., Ltd., trade name "ORGATIX ZC-580")

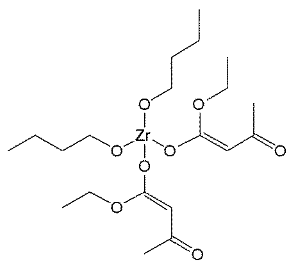

SZ-7

<Surface treatment agent used in Comparative Examples>

SS-1:
1,1,1,3,3,3-hexamethyldisilazane (reagent manufactured by Tokyo Chemical Industry Co., Ltd.)
SS-2:

methyltrichlorosilane (reagent manufactured by Tokyo Chemical Industry Co., Ltd.) SS-3:
vinyltrichlorosilane (reagent manufactured by Tokyo Chemical Industry Co., Ltd.)

[Example 1]

39.4 parts by mass of a vinyl-terminated diphenylsiloxane-dimethylsiloxane copolymer (component (A) in Table 1, "PDV-0541" (trade name) manufactured by Gelest, Inc., weight-average molecular weight: 60,000), 0.6 parts by mass of a methylhydrosiloxane polymer (component (B) in Table 1, "HMS-991" (trade name) manufactured by Gelest, Inc., weight-average molecular weight: 1,600, Si-H equivalent: 67 g/mol), 10.0 parts by mass of a polysiloxane resin (component (C) in Table 1, $(SiO_{4/2})_{1.0}((CH_2=CH)(CH_3)_2SiO_{1/2})_{0.12}((CH_3)_3SiO_{1/2})_{0.75}$), and 50.0 parts by mass of the surface-treated zinc oxide particles (component (D) in Table 2, average primary particle diameter before surface treatment: 35 nm, surface-treated with the surface treatment agent SC-1) prepared in Preparation Example described above were

kneaded with a kneader at a temperature of 23°C for 2 hours to obtain a uniform paste. A platinum catalyst solution (SIP6832.2 manufactured by Gelest, Inc., platinum concentration: 2%) was added at 500 ppm (10 ppm in terms of platinum) to the paste which was then mixed, defoamed under reduced pressure, placed in a 150 mm × 150 mm metal mold, and heat-treated at 60°C for 3 hours to obtain a silicone resin sheet having a thickness of 2.0 mm.

Silicone resin sheets of Examples 2 to 48 and Comparative Examples 1 to 8 were produced in the same manner as the silicone resin sheet of Example 1, except that the compositions shown in Table 2 (summary of Tables 2-1 to 2-3 is referred to as Table 2) shown later were adopted in the production of the silicone resin sheet of Example 1.

[Acoustic velocity]

With regard to the obtained silicone resin sheet having a thickness of 2.0 mm, an acoustic velocity at 25°C was measured using a sing-around acoustic velocity measurement apparatus (manufactured by Ultrasonic Engineering Co., Ltd., trade name "UVM-2 model") in accordance with JIS Z2353 (2003) and evaluated by applying it to the following standard.

<Evaluation standard>

A: acoustic velocity of the silicone resin sheet was less than 840 m/s.
B: acoustic velocity of the silicone resin sheet was 840 m/s or more and less than 870 m/s.
C: acoustic velocity of the silicone resin sheet was 870 m/s or more and less than 900 m/s.
D: acoustic velocity of the silicone resin sheet was 900 m/s or more.

[Adhesion test]

67 parts by mass of an epoxy resin (bisphenol A diglycidyl ether, "jER828" (trade name) manufactured by Mitsubishi Chemical Corporation, epoxy equivalent weight: 189 g/eq) and 33 parts by mass of an amine-based curing agent ("jER Cure ST12" (trade name) manufactured by Mitsubishi Chemical Corporation, amine value: 365 KOHmg/g) were mixed and poured into a mold, and cured at 80°C for 12 hours to obtain an epoxy resin cured sheet having a length of 10 cm, a width of 2 cm, and a thickness of 2 mm.

One end of the epoxy resin cured sheet in the length direction was masked with a Teflon (registered trademark) tape by 1 cm. The entire surface including the masked surface was coated with the paste prepared above to a thickness of 0.4 mm (length of 10 cm and width of 2 cm), and heat-treated at 60°C for 3 hours, and then the masking was removed to obtain a laminated sheet in which the silicone resin sheet was bonded to the epoxy resin sheet. A test piece having a length of 10 cm (including a portion where the paste was not applied) and a width of 1 cm was cut out from the laminated sheet, and using a tensile tester (manufactured by Instron Corporation, product name "3340 type"), a peel strength in a case where the silicone resin sheet was peeled off from the epoxy resin sheet at 90° was measured according to JIS K6864-1 (1999), and the peel strength was evaluated according to the following standard.

<Evaluation standard>

A: peel strength was 20 N/cm or more.
B: peel strength was 15 N/cm or more and less than 20 N/cm.
C: peel strength was 10 N/cm or more and less than 15 N/cm.
D: peel strength was less than 10 N/cm.

[Ozone water resistance test]

The test piece produced above was installed in a flow passage of an ozone water generator (trade name: OWM-10L10P, manufactured by EcoDesign, Inc.), and ozone water having an ozone concentration of 3 ppm was allowed to flow for 3 hours at a flow rate of 1 L/min. Thereafter, the test piece was washed with distilled water and dried at 23°C × 50 %RH (relative humidity) for 24 hours. The same peel strength test as described above was performed on the test piece (after 24 hours of drying). The peel strength of the test piece (before the ozone water treatment) ("peel strength 1") and the peel strength of the test piece (after 24 hours of drying) ("peel strength 2") were substituted in the following expression to calculate a peel strength retention rate before and after the ozone water treatment.

$$\text{Peel strength retention rate (\%)} = 100 \times \text{peel strength 2/peel strength 1}$$

The peel strength retention rate was evaluated according to the following evaluation standard.

<Evaluation standard>

A: peel strength retention rate was 90% or more.
B: peel strength retention rate was 75% or more and less than 90%.
C: peel strength retention rate was 50% or more and less than 75%.
D: peel strength retention rate was less than 50%.

[Table 2-1]

| | | | EX1 | EX2 | EX3 | EX4 | EX5 | EX6 | EX7 | EX8 | EX9 | EX10 | EX11 | EX12 |
|---|---|---|---|---|---|---|---|---|---|---|---|---|---|---|
| Composition | Component (A) | Type | A-1 | A-1 | A-1 | A-1 | A-1 | A-1 | A-1 | A-1 | A-1 | A-1 | A-1 | A-1 |
| | | Content [part by mass] | 39.4 | 39.4 | 39.4 | 39.4 | 39.4 | 39.4 | 39.4 | 39.4 | 39.4 | 39.4 | 39.4 | 39.4 |
| | Component (B) | Type | B-1 | B-1 | B-1 | B-1 | B-1 | B-1 | B-1 | B-1 | B-1 | B-1 | B-1 | B-1 |
| | | Content [part by mass] | 0.6 | 0.6 | 0.6 | 0.6 | 0.6 | 0.6 | 0.6 | 0.6 | 0.6 | 0.6 | 0.6 | 0.6 |
| | Component (C) | Type | C-1 | C-1 | C-1 | C-1 | C-1 | C-1 | C-1 | C-1 | C-1 | C-1 | C-1 | C-1 |
| | | Content [part by mass] | 10.0 | 10.0 | 10.0 | 10.0 | 10.0 | 10.0 | 10.0 | 10.0 | 10.0 | 10.0 | 10.0 | 10.0 |
| | Component (D) | Type | D-1 | D-2 | D-3 | D-4 | D-5 | D-6 | D-7 | D-8 | D-9 | D-10 | D-11 | D-12 |
| | | Average primary particle diameter [nm] | 35 | 35 | 35 | 35 | 35 | 35 | 35 | 35 | 35 | 35 | 35 | 35 |
| | | Surface treatment agent (S) | SC-1 | SC-2 | SC-3 | SC-4 | SC-5 | SC-6 | SA-1 | SA-2 | SM-1 | SM-2 | SI-1 | SI-2 |
| | | Treated amount [php] | 30 | 30 | 30 | 30 | 30 | 30 | 30 | 30 | 30 | 30 | 30 | 30 |
| | | Content [part by mass] | 50.0 | 50.0 | 50.0 | 50.0 | 50.0 | 50.0 | 50.0 | 50.0 | 50.0 | 50.0 | 50.0 | 50.0 |
| Evaluation | Acoustic velocity | | C | C | B | B | B | B | B | B | A | A | A | A |
| | Adhesiveness | | C | C | C | C | C | C | A | B | C | C | A | A |
| | Ozone water resistance | | C | C | C | C | C | C | C | C | C | C | C | C |

| | | | EX13 | EX14 | EX15 | EX16 | EX17 | EX18 | EX19 | EX20 | EX21 | EX22 | EX23 | EX24 |
|---|---|---|---|---|---|---|---|---|---|---|---|---|---|---|
| Composition | Component (A) | Type | A-1 | A-1 | A-1 | A-1 | A-1 | A-1 | A-1 | A-1 | A-1 | A-1 | A-1 | A-1 |
| | | Content [part by mass] | 39.4 | 39.4 | 39.4 | 39.4 | 39.4 | 39.4 | 39.4 | 39.4 | 39.4 | 39.4 | 39.4 | 39.4 |
| | Component (B) | Type | B-1 | B-1 | B-1 | B-1 | B-1 | B-1 | B-1 | B-1 | B-1 | B-1 | B-1 | B-1 |
| | | Content [part by mass] | 0.6 | 0.6 | 0.6 | 0.6 | 0.6 | 0.6 | 0.6 | 0.6 | 0.6 | 0.6 | 0.6 | 0.6 |
| | Component (C) | Type | C-1 | C-1 | C-1 | C-1 | C-1 | C-1 | C-1 | C-1 | C-1 | C-1 | C-1 | C-1 |
| | | Content [part by mass] | 10.0 | 10.0 | 10.0 | 10.0 | 10.0 | 10.0 | 10.0 | 10.0 | 10.0 | 10.0 | 10.0 | 10.0 |
| | Component (D) | Type | D-13 | D-14 | D-15 | D-16 | D-17 | D-18 | D-19 | D-20 | D-21 | D-22 | D-23 | D-24 |
| | | Average primary particle diameter [nm] | 35 | 35 | 35 | 35 | 35 | 35 | 35 | 35 | 35 | 35 | 35 | 35 |
| | | Surface treatment agent (S) | ST-1 | ST-2 | ST-3 | SL-1 | SL-2 | SL-3 | SL-4 | SL-5 | SZ-1 | SZ-2 | SZ-3 | SZ-4 |
| | | Treated amount [php] | 30 | 30 | 30 | 30 | 30 | 30 | 30 | 30 | 30 | 30 | 30 | 30 |
| | | Content [part by mass] | 50.0 | 50.0 | 50.0 | 50.0 | 50.0 | 50.0 | 50.0 | 50.0 | 50.0 | 50.0 | 50.0 | 50.0 |
| Evaluation | Acoustic velocity | | C | C | C | B | A | A | A | A | B | B | B | B |
| | Adhesiveness | | C | C | C | A | A | A | A | A | B | B | A | B |
| | Ozone water resistance | | C | C | C | B | A | A | A | A | B | B | B | B |

[Table 2-2]

| | | | EX25 | EX26 | EX27 | EX28 | EX29 | EX30 | EX31 | EX32 | EX33 | EX34 | EX35 | EX36 |
|---|---|---|---|---|---|---|---|---|---|---|---|---|---|---|
| Composition | Component (A) | Type | A-1 | A-1 | A-1 | A-1 | A-1 | A-1 | A-1 | A-1 | A-1 | A-2 | A-3 | A-4 |
| | | Content [part by mass] | 39.4 | 39.4 | 39.4 | 39.4 | 39.4 | 39.4 | 39.4 | 39.4 | 39.4 | 39.2 | 39.7 | 39.6 |
| | Component (B) | Type | B-1 | B-1 | B-1 | B-1 | B-1 | B-1 | B-1 | B-1 | B-1 | B-1 | B-1 | B-1 |
| | | Content [part by mass] | 0.6 | 0.6 | 0.6 | 0.6 | 0.6 | 0.6 | 0.6 | 0.6 | 0.6 | 0.8 | 0.3 | 0.4 |
| | Component (C) | Type | C-1 | C-1 | C-1 | C-1 | C-1 | C-1 | C-1 | C-1 | C-1 | C-1 | C-1 | C-1 |
| | | Content [part by mass] | 10.0 | 10.0 | 10.0 | 10.0 | 10.0 | 10.0 | 10.0 | 10.0 | 10.0 | 10.0 | 10.0 | 10.0 |
| | Component (D) | Type | D-25 | D-26 | D-27 | D-28 | D-29 | D-30 | D-31 | D-32 | D-33 | D-19 | D-19 | D-19 |
| | | Average primary particle diameter [nm] | 35 | 35 | 35 | 20 | 60 | 200 | 250 | 35 | 35 | 35 | 35 | 35 |
| | | Surface treatment agent (S) | SZ-5 | SZ-6 | SZ-7 | SL-4 | SL-4 | SL-4 | SL-4 | SL-4 | SL-4 | SL-4 | SL-4 | SL-4 |
| | | Treated amount [php] | 30 | 30 | 30 | 30 | 30 | 30 | 30 | 10 | 50 | 30 | 30 | 30 |
| | | Content [part by mass] | 50.0 | 50.0 | 50.0 | 50.0 | 50.0 | 50.0 | 50.0 | 50.0 | 50.0 | 50.0 | 50.0 | 50.0 |
| Evaluation | Acoustic velocity | | B | B | A | A | A | A | B | B | A | A | A | A |
| | Adhesiveness | | B | A | A | A | A | B | C | C | B | B | C | B |
| | Ozone water resistance | | B | B | B | A | B | C | C | C | B | A | C | C |

| | | | EX37 | EX38 | EX39 | EX40 | EX41 | EX42 | EX43 | EX44 | EX45 | EX46 | EX47 | EX48 |
|---|---|---|---|---|---|---|---|---|---|---|---|---|---|---|
| Composition | Component (A) | Type | A-5 | A-6 | A-7 | A-1 | A-1 | A-1 | A-1 | A-1 | A-1 | A-1 | A-1 | A-1 |
| | | Content [part by mass] | 39.5 | 39.4 | 38.5 | 39.0 | 39.4 | 39.4 | 39.4 | 44.3 | 32.6 | 59.2 | 49.5 | 29.6 |
| | Component (B) | Type | B-1 | B-1 | B-1 | B-2 | B-1 | B-1 | B-1 | B-1 | B-1 | B-1 | B-1 | B-1 |
| | | Content [part by mass] | 0.5 | 0.6 | 1.5 | 1.0 | 0.6 | 0.6 | 0.6 | 0.7 | 0.4 | 0.8 | 0.5 | 0.4 |
| | Component (C) | Type | C-1 | C-1 | C-1 | C-1 | C-2 | C-3 | C-4 | C-1 | C-1 | C-1 | C-1 | C-1 |
| | | Content [part by mass] | 10.0 | 10.0 | 10.0 | 10.0 | 10.0 | 10.0 | 10.0 | 6.0 | 17.0 | 10.0 | 10.0 | 10.0 |
| | Component (D) | Type | D-19 | D-19 | D-19 | D-19 | D-19 | D-19 | D-19 | D-19 | D-19 | D-19 | D-19 | D-19 |
| | | Average primary particle diameter [nm] | 35 | 35 | 35 | 35 | 35 | 35 | 35 | 35 | 35 | 35 | 35 | 35 |
| | | Surface treatment agent (S) | SL-4 | SL-4 | SL-4 | SL-4 | SL-4 | SL-4 | SL-4 | SL-4 | SL-4 | SL-4 | SL-4 | SL-4 |
| | | Treated amount [php] | 30 | 30 | 30 | 30 | 30 | 30 | 30 | 30 | 30 | 30 | 30 | 30 |
| | | Content [% by mass] | 50.0 | 50.0 | 50.0 | 50.0 | 50.0 | 50.0 | 50.0 | 50.0 | 50.0 | 300 | 40.0 | 60.0 |
| Evaluation | Acoustic velocity | | A | A | A | A | A | A | A | A | B | C | B | A |
| | Adhesiveness | | B | B | C | A | B | A | B | B | A | B | A | A |
| | Ozone water resistance | | C | C | C | A | A | A | B | B | A | B | A | B |

[Table 2-3]

| | | | CEX1 | CEX2 | CEX3 | CEX4 | CEX5 | CEX6 | CEX7 | CEX8 |
|---|---|---|---|---|---|---|---|---|---|---|
| Composition | Component (A) | Type | A-1 | A-1 | A-1 | A-1 | A-1 | A-1 | A-1 | A-1 |
| | | Content [part by mass] | 98.8 | 88.9 | 49.4 | 39.4 | 39.4 | 39.4 | 39.4 | 39.0 |
| | Component (B) | Type | B-1 | B-1 | B-1 | B-1 | B-1 | B-1 | B-1 | B-2 |
| | | Content [part by mass] | 1.2 | 1.1 | 0.6 | 0.6 | 0.6 | 0.6 | 0.6 | 1.0 |
| | Component (C) | Type | - | C-1 | - | C-1 | C-1 | C-1 | C-1 | C-1 |
| | | Content [part by mass] | | 10.0 | | 10.0 | 10.0 | 10.0 | 10.0 | 10.0 |
| | Component (D) | Type | | - | D-19 | D-37 | D-34 | D-35 | D-36 | Q-7 |
| | | Average primary particle diameter [nm] | | | 35 | 350 | 35 | 35 | 35 | 30 |
| | | Surface treatment agent (S) | | | SL-4 | SL-4 | SS-1 | SS-2 | SS-3 | MTMS, HMDS |
| | | Treated amount [php] | | | 30 | 30 | 30 | 30 | 30 | - |
| | | Content [part by mass] | | | 50.0 | 50.0 | 50.0 | 50.0 | 50.0 | 50.0 |
| Evaluation | Acoustic velocity | | D | D | A | C | C | C | C | D |
| | Adhesiveness | | D | D | A | C | D | D | D | D |
| | Ozone water resistance | | D | D | D | D | D | D | D | D |

<Notes of table>

"EX": Example
"CEX": Comparative Example
"php": part by mass of surface treatment agent with respect to 100 parts by mass of zinc oxide particles
"Average primary particle diameter": in a case of surface-treated zinc oxide particles (D-1 to D-37), average primary particle diameter of the zinc oxide particles before the surface treatment, and in a case of surface-treated silica (Q-7), average primary particle diameter of the surface-treated product; The surface-treated zinc oxide particles (D-1 to D-37) all had an average primary particle diameter in a surface-treated state of more than 20 nm and less than 300 nm. In addition, the surface-treated zinc oxide particles (D-30) had an average primary particle diameter in a surface-treated state of 250 nm or less, and the surface-treated zinc oxide particles (D-29) had an average primary particle diameter in a surface-treated state of 100 nm or less.

[Polysiloxane (A) having vinyl group]

(A-1): vinyl-terminated diphenylsiloxane-dimethylsiloxane copolymer (manufactured by Gelest, Inc., trade name "PDV-0541", weight-average molecular weight: 60,000, amount of diphenylsiloxane: 5 mol%)
(A-2): vinyl-terminated diphenylsiloxane-dimethylsiloxane copolymer (manufactured by Gelest, Inc., trade name "PDV-0535", weight-average molecular weight: 47,500, amount of diphenylsiloxane: 5 mol%)
(A-3): vinyl-terminated polydimethylsiloxane (manufactured by Gelest, Inc., trade name "DMS-V52", weight-average molecular weight: 155,000)
(A-4): vinyl-terminated polydimethylsiloxane (manufactured by Gelest, Inc., trade name "DMS-V46", weight-average molecular weight: 117,000)
(A-5): vinyl-terminated polydimethylsiloxane (manufactured by Gelest, Inc., trade name "DMS-V42", weight-average molecular weight: 72,000)
(A-6): vinyl-terminated polydimethylsiloxane (manufactured by Gelest, Inc., trade name "DMS-V41", weight-average molecular weight: 62,700)
(A-7): vinyl-terminated polydimethylsiloxane (manufactured by Gelest, Inc., trade name "DMS-V31", weight-average molecular weight: 28,000)

[Polysiloxane (B) having Si-H group]

(B-1):

polymethylhydroxysiloxane (manufactured by Gelest, Inc., trade name "HMS-991", weight-average molecular weight: 1,600, methylhydroxysiloxy unit: 100 mol%, Si-H equivalent: 67 g/mol)
(B-2):
methylhydrosiloxane-phenylmethylsiloxane copolymer (manufactured by Gelest, Inc., trade name "HPM-502", weight-average molecular weight: 4,500, methylhydroxysiloxy unit: 47 mol%, Si-H equivalent: 165 g/mol)

[Polysiloxane resin (C)]

(C-1):

polysiloxane resin represented by the following average compositional formula (M/Q = 0.87, see JP2014-80546A)

$$(SiO_{4/2})_{1.0}((CH_2{=}CH)(CH_3)_2SiO_{1/2})_{0.12}((CH_3)_3SiO_{1/2})_{0.75}$$

a kinematic viscosity of a 50% by mass xylene solution of the polysiloxane resin (C-1) was 3.0 $mm^2/s$.

(C-2):

polysiloxane resin represented by the following average compositional formula (M/Q = 1.75, see JP2014-80546A)

$$(sio_{4/2})_{1.0}((CH_2=CH)(CH_3)_2Si_{1/2})_{0.25}((CH_3)_3SiO_{1/2})_{1.5}$$

a kinematic viscosity of a 50% by mass xylene solution of the polysiloxane resin (C-2) was 1.5 $mm^2/s$.

(C-3):

polysiloxane resin represented by the following average compositional formula (M/Q = 1.06, see JP1994-331079A (JP-H7-331079A))

$$(sio_{4/2})_{1.0}((CH_2=CH)(CH_3)_2\ SiO_{1/2})_{0.12}((CH_3)_3SiO_{1/2})_{0.94}$$

a kinematic viscosity of a 50% by mass xylene solution of the polysiloxane resin (C-3) was 3.5 $mm^2/s$.

(C-4):

polysiloxane resin represented by the following average compositional formula (M/Q = 0.62)

$$(SiO_{4/2})_{1.0}((CH_3)SiO_{3/2})_{0.25}((CH_2=CH)(CH_3)_2SiO_{1/2})_{0.12}((CH_3)_3SiO_{1/2})_{0.50}$$

a kinematic viscosity of a 50% by mass xylene solution of the polysiloxane resin (C-4) was 3.3 $mm^2/s$.

From Table 2, the following was found.

All test results of the silicone resin sheet of Comparative Example 1, which was produced without using the component (C) and the component (D), the silicone resin sheet of Comparative Example 2, which was produced without using the component (D), and the silicone resin sheet of Comparative Example 8, which was produced using the surface-treated silica instead of the component (D), were unacceptable.

In the silicone resin sheet of Comparative Example 3, which was produced without using the component (C), the ozone water resistance was unacceptable.

In the silicone resin sheet of Comparative Example 4, which was produced using the zinc oxide particles having an average primary particle diameter exceeding the upper limit specified in the present invention, the ozone water resistance was unacceptable.

In the silicone resin sheets of Comparative Examples 5 to 7, which were produced without using the surface treatment agent specified in the present invention, the ozone water resistance was unacceptable.

On the other hand, it was found that the silicone resin sheet produced using the composition according to the embodiment of the present invention had a low acoustic velocity, was difficult to peel off from the acoustic matching layer, and had excellent ozone water resistance.

It is considered that the reason why the silicone resin sheet produced using the aluminum alkoxide compound and the zirconium alkoxide compound as the surface treatment agent had excellent ozone water resistance is that first ionization energies of aluminum and zirconium are relatively low.

The present invention has been described with the embodiments thereof, any details of the description of the present invention are not limited unless described otherwise, and it is obvious that the present invention is widely construed without departing from the gist and scope of the present invention described in the accompanying claims.

The present application claims the priority of JP2021-186567 filed in Japan on November 16, 2021, the contents of which are incorporated herein by reference, as a part of the description of the present specification.

Explanation of References

1: acoustic lens
2: acoustic matching layer
3: piezoelectric element layer
4: backing material

7: housing
9: cord
10: ultrasound probe

## Claims

1. A composition for an acoustic lens, comprising the following components (A) to (D):

(A) a linear polysiloxane having a vinyl group;
(B) a linear polysiloxane having two or more Si-H groups in a molecular chain;
(C) a polysiloxane resin; and
(D) zinc oxide particles having an average primary particle diameter of more than 10 nm and less than 300 nm, and surface-treated with at least one surface treatment agent of a silane compound, an aluminum alkoxide compound, a zirconium alkoxide compound, or a titanium alkoxide compound,

provided that the silane compound has at least one of a hydroxy group directly bonded to a silicon atom or an alkoxy group directly bonded to the silicon atom.

2. The composition for an acoustic lens according to claim 1, wherein a content of the component (D) is 25 to 70 parts by mass in 100 parts by mass of a total content of the components (A) to (D).

3. The composition for an acoustic lens according to claim 1 or 2, wherein, in 100 parts by mass of a total content of the components (A) to (D), a content of the component (A) is 20 to 70 parts by mass, a content of the component (B) is 0.1 to 20 parts by mass, and a content of the component (C) is 0.1 to 50 parts by mass.

4. The composition for an acoustic lens according to any one of claims 1 to 3,

wherein the silane compound includes at least one of an aminosilane compound, a mercaptosilane compound, an isocyanatosilane compound, or a thiocyanatosilane compound,
provided that the aminosilane compound, the mercaptosilane compound, the isocyanatosilane compound, and the thiocyanatosilane compound have at least one of a hydroxy group directly bonded to a silicon atom or an alkoxy group directly bonded to the silicon atom.

5. The composition for an acoustic lens according to any one of claims 1 to 4, wherein a content of the surface treatment agent in the component (D) is 1 to 100 parts by mass with respect to 100 parts by mass of the zinc oxide particles constituting the component (D).

6. The composition for an acoustic lens according to any one of claims 1 to 5, wherein the component (A) has a phenyl group.

7. The composition for an acoustic lens according to any one of claims 1 to 6, wherein a weight-average molecular weight of the component (A) is 20,000 to 200,000.

8. The composition for an acoustic lens according to claim 7, wherein the weight-average molecular weight is 40,000 to 150,000.

9. The composition for an acoustic lens according to any one of claims 1 to 8,

wherein the component (C) is a polysiloxane resin which includes an $R_3SiO_{1/2}$ unit and an $SiO_{4/2}$ unit and has at least two vinyl groups in one molecule, and a molar ratio of the $R_3SiO_{1/2}$ unit to the $SiO_{4/2}$ unit is 0.6 to 1.2, provided that R represents a monovalent hydrocarbon group.

10. The composition for an acoustic lens according to claim 9, wherein the component (C) consists of the $RSiO_{1/2}$ unit and the $SiO_{4/2}$ unit.

**11.** The composition for an acoustic lens according to any one of claims 1 to 10,
wherein the average primary particle diameter of the zinc oxide particles constituting the component (D) is 10 to 200 nm.

**12.** The composition for an acoustic lens according to any one of claims 1 to 11, further comprising:
at least one of platinum or a platinum compound in an amount of 0.00001 to 0.01 parts by mass with respect to 100 parts by mass of a total content of the components (A) to (D).

**13.** An acoustic lens obtained by curing the composition for an acoustic lens according to any one of claims 1 to 12.

**14.** An acoustic wave probe comprising:
the acoustic lens according to claim 13.

**15.** An ultrasound probe comprising:
the acoustic lens according to claim 13.

**16.** An acoustic wave measurement apparatus comprising:
the acoustic wave probe according to claim 14.

**17.** An ultrasound diagnostic apparatus comprising:
the acoustic wave probe according to claim 14.

**18.** A photoacoustic wave measurement apparatus comprising:
the acoustic lens according to claim 13.

**19.** An ultrasonic endoscope comprising:
the acoustic lens according to claim 13.

**20.** A method for manufacturing an acoustic wave probe, comprising:
forming an acoustic lens using the composition for an acoustic lens according to any one of claims 1 to 12.

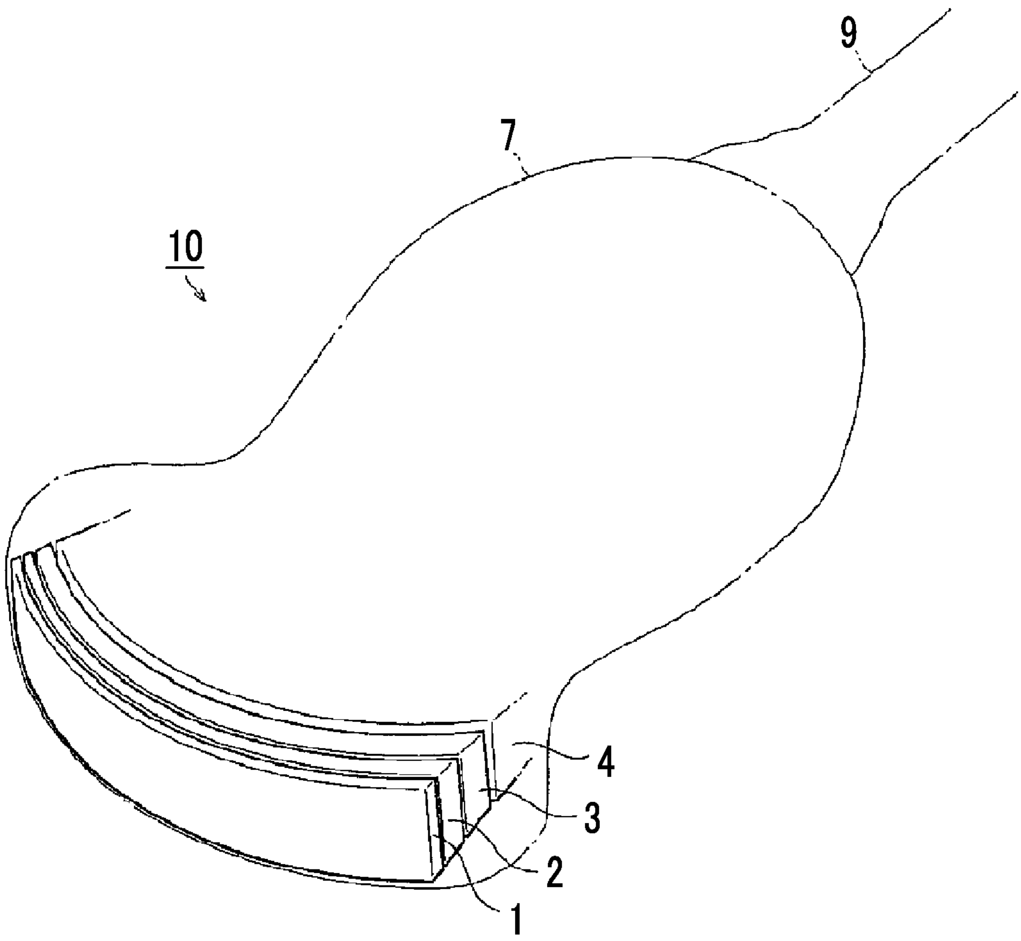
9
7
10
4
3
2
1

## INTERNATIONAL SEARCH REPORT

International application No.
**PCT/JP2022/041674**

**A. CLASSIFICATION OF SUBJECT MATTER**

***A61B 8/00***(2006.01)i; ***A61B 8/12***(2006.01)i; ***A61B 8/13***(2006.01)i; ***C08K 9/00***(2006.01)i; ***C08L 83/04***(2006.01)i; ***C08L 83/05***(2006.01)i; ***C08L 83/07***(2006.01)i
FI: A61B8/00; A61B8/12; A61B8/13; C08K9/00; C08L83/04; C08L83/05; C08L83/07

According to International Patent Classification (IPC) or to both national classification and IPC

**B. FIELDS SEARCHED**

Minimum documentation searched (classification system followed by classification symbols)

A61B8/00-A61B8/15; C08K3/00-C08K13/08; C08L1/00-C08L101/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2021/044823 A1 (FUJIFILM CORP.) 11 March 2021 (2021-03-11) abstract, paragraphs [0021]-[0059], [0062], [0136], [0139], [0141]-[0144] | 1-20 |
| A | JP 2014-080546 A (SHIN-ETSU CHEMICAL CO., LTD.) 08 May 2014 (2014-05-08) entire text, all drawings | 1-20 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 January 2023** | **17 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/041674**

| Patent document cited in search report | Publication date (day/month/year) | Patent family member(s) | Publication date (day/month/year) |
|---|---|---|---|
| WO 2021/044823 A1 | 11 March 2021 | US 2022/0172701 A1<br>abstract, paragraphs [0088]-[0149], [0153], [0343], [0349], [0355]-[0361]<br>EP 4026872 A1<br>CN 114269860 A | |
| JP 2014-080546 A | 08 May 2014 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021044823 A
- WO 2017130890 A
- JP 2003169802 A
- JP 2013202050 A
- JP 2013188465 A
- JP 2011071842 A
- JP 2006157320 A
- JP 2013158435 A
- JP 2008311700 A
- JP 2014080546 A
- JP 6331079 A
- JP H7331079 A
- JP 2021186567 A